# EUROPEAN PATENT SPECIFICATION

(11) **EP 0 512 390 B1**
(45) Date of publication and mention of the grant of the patent: **30.10.1996**
(21) Application number: 92107291.4
(22) Date of filing: 29.04.1992
(51) Int. Cl.: G01N 33/558, G01N 33/52, G01N 33/58

(54) **Test strip for immunoassay, assay method using the same and immunoassay device**
Teststreifen für Immunoassay, Testverfahren zu seiner Verwendung und Gerät für Immunotests
Elément d'analyse pour l'essai immunologique, procédé d'analyse l'utilisant et dispositif pour les essais immunologiques

(30) Priority: 02.05.1991 JP 128275/91; 31.10.1991 JP 311352/91
(43) Date of publication of application: 11.11.1992
(73) Proprietor: FUJIREBIO Inc., Shinjuku-ku, Tokyo 163-07 (JP)
(72) Inventor: Ashihara, Yoshihiro, Fuchu-shi, Tokyo (JP); Hasegawa, Akira, Fussa-shi, Tokyo (JP); Ono, Masaaki, Tanashi-shi, Tokyo (JP); Kaneko, Hideto, Kunitachi-shi, Tokyo (JP); Koiwai, Kazunori, Sagamihara-shi, Kanagawa-ken (JP)
(74) Representative: Hansen, Bernd, Dr. Dipl.-Chem.

(56) References cited:
- EP-A- 0 262 328
- WO-A-88/08536
- US-A- 4 956 275

## Description

### BACKGROUND OF THE INVENTION

This invention relates a test strip for immunoassay, an assay method using the same and an immunoassay device, which can assay an antigen or an antibody in a sample simply and easily.

As one of immunoassay methods, a device using the method in which an absorption material is used, one end of which is added a liquid layer incorporated therein a developing solution containing a coloring substrate, and after adding a specimen to the absorption material, the liquid layer is broken to develop the developing solution and coloring due to the reaction between an enzyme and the substrate in the absorption material is detected is called "dip stick". The device has been known as a simple device which requires no washing operation nor an operation of adding a substrate, etc. during measurement as compared to the conventional immunoassay method (see, for example, Japanese Provisional Patent Publications No. 145459/1986 (which corresponds to DE P 34 45 816.6), No. 501595/1988 (which corresponds to WO 87/02774) and No. 503439/1989 (which corresponds to WO 88/08536)).

However, either of the devices involves the problems that they employ the method of measuring a colored amount whereby sensitivity is low so that it cannot be used for a measurement in which high sensitivity is required, it colors due to the reaction of an enzyme and a substrate during development and increase in background causes so that accurate detection of a minor component cannot be effected, and formation of a dye takes much times so that they require a long measurement time from initiation of the measurement to the time at which the results are obtained. Further, in these devices, one dip stick is required for one measurement item and they cannot deal with plural items at once which require high sensitivity and specific reactions.

It is also important to analyze living body components or drugs contained in blood or urine for diagnosis of conditions of disease and judgement of progress after therapy, whereby it fulfills an important role in the field of clinical inspection. Such a clinical inspection is preferably carried out simply and easily in a clinic, and when self inspection is to be carried out in a home, it is desired to carry out easily and rapidly without any specific equipments.

In case where an object to be analyzed is one having enzymatic activity (e.g. amylase) or one which can be a substrate for a specific enzyme (e.g. glucose in urine), a filter paper on a strip to which a reaction reagent is impregnated is dipped in a sample (such as urine, blood, etc.) and the presence or absence of an enzyme to be tested can be measured by coloring. There is a dip stick type one utilizing the above reaction.

In case where an object to be analyzed do not participate directly in a coloring reaction, its analysis can be carried out by using an enzyme immunoassay method (EIA method). The EIA method is a method in which an antigen-antibody reaction is traced by using an enzyme activity as a label and an amount of an antigen or antibody is determined therefrom. Its measurement systems are divided into many methods according to the difference in an object to be measured, a labelled substance, a form of a reaction of antigen-antibody at measurement, and the presence or absence of B/F separation after the antigen-antibody reaction or a method thereof. Anyway, for their analyses, preparations of various kinds of reagents are required so that various analytical elements to which necessary reagents (antibody, enzyme-labelled antigen, enzyme substrate, coloring reagent, etc.) depending on the system of a reaction are previously provided have been proposed.

For example, in Japanese Provisional PCT Patent Publication No. 503439/1989 (WO88/08536), an assay device which can be used simply and easily without requiring any specific pretreatment is disclosed.

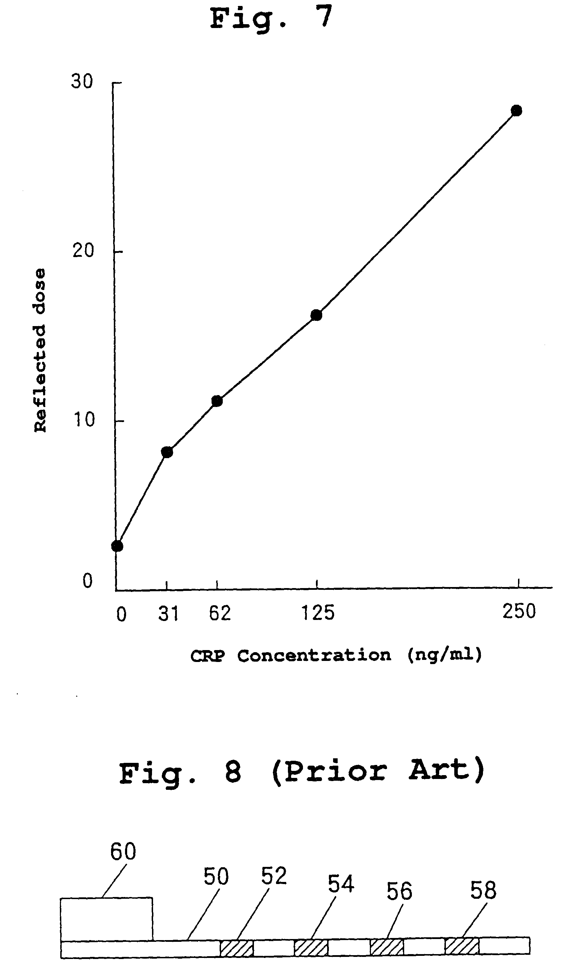

This is a device, as shown in Fig. 8, in which on a strip piece 50 of a water-absorbable material (e.g. a filter paper) which can transport a developing solution by capillarity were provided a signal preventing zone 52, a sample receiving zone 54, a reagent zone 56 and an indicator zone 58, while a developing solution reservoir 60 is provided at the one end of the strip piece 50. The developing solution reservoir 60 contains a substrate of a labelled enzyme and a coloring agent, and a signal preventing agent (e.g. an enzyme inhibitor) which prevents coloring due to an enzyme reaction is impregnated in the signal preventing zone 52. Explanation is made when a sample is an antigen. In the reagent zone 56, an enzyme-labelled antigen is impregnated, and in the indicator zone 58, an immobilized antibody is contained. After dotting a sample solution to the sample receiving zone 54, when the strip piece 50 is dipped in the developing reservoir, the substrate solution transfers and diffuses the antigen in the sample receiving zone 54 and the enzyme-labelled antigen in the reagent zone 56 to the indicator zone 58. As the results, the enzyme-labelled antigen and the sample antigen competitively react with the immobilized antibody in the indicator zone 58. Free antigens and labelled antigens which are not bound to the immobilized antibody are washed out from the indicator zone 58 by the substrate solution subsequently supplied, and in the indicator zone 58, the labelled antigen is remained in an amount which is in inverse proportion to an amount of the antigen to be tested. And the amount of the labelled antigen immobilized is determined by color forming reaction of the labelled enzyme and the substrate in the substrate solution.

At this time, the reaction between the antigen-labelled enzyme and the substrate in the substrate solution during developing the strip piece 50 is restrained by the signal preventing agent supplied from the signal preventing zone 52. Accompanying with diffusion of the substrate solution, the labelled antigen, the signal preventing agent and the antigen to be tested which are impregnated in respective zones are each washed out to move while positioning at the top of the diffused substrate solution. Accordingly, until the top of the developing solution reaches to the indicator zone 58, the reaction of the labelled enzyme is restrained. However, once the labelled antigen is immobilized by the immobilized antibody, the signal inhibiting agent is washed out with free labelled antigens by the substrate solution subsequently supplied, so that the enzyme of the labelled-antigen is subjected to color forming reaction at this time.

When an antigen to be tested is a polyvalent antigen, a labelled antibody may be used in place of the labelled antigen. In this case, the antigen to be tested is detected in a form that it is sandwiched between the immobilized antibody and the labelled antibody (so-called sandwich method).

Thus, in the assay device of the prior art, the presence or absence and an amount of an antigen (or an antibody) to be tested can be measured easily by measuring a color at the indicator zone.

However, in the assay device, an enzyme inhibitor is required to inhibit the reaction of the labelled enzyme which is moving by the substrate solution.

Also, since an antigen, particularly a low molecular weight antigen may sometimes change in its antigenic property due to labelled enzyme, the sandwich method using an antibody by labelling with an enzyme is generally and widely applied to this kind of the immunoassay device (or element). However, this assay device is to move the antigen to be tested and the labelled antibody to an indicator zone with a single substrate solution simultaneously and to effect binding reaction so that the sandwich method which can be applied to the above is only a one step method. However, in the one step method, when the antigen to be tested exists in an excessive amount, an antigen-immobilized antibody bound product and an antigen-labelled antibody bound product are also formed in larger amounts and they cannot be bound to each other. That is, when an excessive amount of the antigen is present, a bound product of the immobilized antibody-antigen-labelled enzyme is not formed in proportion to an amount of the antigen so that a measured value gives apparently lower one. Thus, when an antigen concentration becomes a higher value exceeding a predetermined concentration (inhibitory zone concentration: prozone), stoichiometric antigen-antibody bound reaction does not occur so that a calibration curve shows diphasic property. Such a prozone effect becomes a cause of making a mistake in judgement whether obtained values correspond to high antigen concentration or to low antigen concentration. Therefore, judgement for an accurate antigen concentration must be done by diluting an antigen to be tested to several concentrations and comparing the respective measured values. It is complicated that a sample to be tested is diluted or an assay is effected with respect to the diluted plural number of samples and it also increases a cost for inspection.

Further, the following problems exist. When a material to be tested is an antibody, an enzyme-labelled second antibody is sometimes used. The second antibody is an antibody against an antibody (generally IgG), and is widely used since labelling enzyme is relatively easy and it can be used for general purpose such as measurement of various antibodies. The second antibody binds, in addition to the antibody to be tested, to non specific IgG existing in a sample solution. Accordingly, it must take step wise procedure in which the antibody to be tested is previously bound to an immobilized antigen, and after removing existing IgG by washing operation, the antigen to be tested is contacted with the enzyme-labelled second antibody. Thus, in the aforesaid assay device in which all the antigen and the antibody are subjected to bound reaction with one step, the assay method using the second antibody cannot be applied to.

### SUMMARY OF THE INVENTION

The present invention has been accomplished under the above situation and an object thereof is to provide a test strip for immunoassay and an assay method using the same, which can assay an antigen or an antibody in a sample simply and readily.

Another object of the present invention is to provide an immunoassay device which is not required to use an enzyme inhibitor and can effect high sensitivity measurement simply and easily.

Also, a further object of the present invention is to provide an immunoassay device which does not cause diphasic reaction due to the prozone effect, does not require complicated diluting operation and causing increase in costs for inspection.

A fourth object of the present invention is to provide an immunoassay device which is suitable for an assay method using a second antibody.

A fifth object of the present invention is to provide an immunoassay method using the immunoassay device.

The above objects of the present invention can be accomplished by a test strip to which a reagent zone for inhibiting light emission and a reagent zone for initiating light emission are provided and an immunoassay device to which at least one washing solution reservoir for washing and preventing interference between reagents is provided on a matrix which is capable of transferring a solution by capillarity.

The present inventors have intensively studied the novel immunoassay device and an assay method using the same, and as the results, they have found that in a test strip for simple and easy immunoassay having a developing solution reservoir at one end to a longitudinal direction of a developing material having capillarity, by adding a light emitting substrate to said developing solution, and providing a reagent zone inhibiting light emission, a reagent zone initiating light emission, an enzyme-labelling reagent zone, a specimen dotting zone and an emitted light detecting zone to a longitudinal direction of said developing material; and also, in a method of detecting a specimen using a test strip comprising a developing solution reservoir which contains a light emitting substrate and provided at one end to a longitudinal direction of a developing material having capillarity, and being provided a plural number of reagent zones, an enzyme-labelling reagent zone, a specimen dotting zone and an emitted light detecting zone to the test strip, and the specimen being detected by moving, during respective zones, said developing solution in the test strip by capillarity, by making one of the reagent zone inhibiting light emission and light emission being initiated in one of the reagent zone, whereby accomplished the present invention.

That is, an assay method of the present invention comprises dotting a specimen to a developing material, developing a developing solution containing a substrate solution to the developing material, causing an antigen-antibody reaction during passing through each reagent zone and binding an enzyme-labelled antibody to a detecting zone. By detecting an emitted amount occurred by a reaction between an enzyme and a substrate at the detecting zone, an amount of an object to be measured in the specimen can be measured. The method is that, at the measurement, a reagent zone for inhibiting light emission occurred by the reaction between the enzyme and the substrate and a reagent zone for initiating light emission are provided to.

Also, the above objects of the present invention can be accomplished by an immunoassay device provided at least one washing solution reservoir for washing and for preventing interference between reagents to each other to a matrix which can transfer a solution by capillarity.

Further, in one of the preferred embodiments of the present invention, an immunoassay device for assaying an antigen to be tested in a sample solution is provided by the following constituents:
(a) a matrix which can transfer a solution by capillarity,
(b) a specimen dotting zone provided in the above matrix, to which a sample solution containing an antigen to be tested is dotted,
(c) a labelling reagent zone containing an antigen or antibody which is labelled by a labelling enzyme and provided in the matrix at a position separate from the specimen dotting zone,
(d) a detecting zone containing an immobilized antibody and provided in the matrix at a position downstream, with respect to the direction of solution transfer, of the specimen dotting zone and the labelling reagent zone,
(e) a substrate solution reservoir containing a substrate of the labelling enzyme and provided in the matrix at a position upstream, with respect to the direction of solution transfer, of the specimen dotting zone and the labelling reagent zone, and
(f) a washing solution reservoir provided in the matrix at a position upstream, with respect to the direction of solution transfer, of the labelling reagent zone and downstream of the substrate solution reservoir.

In the above embodiment, a washing solution reservoir is provided upstream, with respect to the direction of solution transfer, of a labelling reagent zone, and prior to development and movement of a substrate solution from a substrate solution reservoir, the washing solution in the washing solution reservoir is supplied on a matrix whereby an antigen to be tested and a labelled antigen (or a labelled antibody) are transferred and moved to a detecting zone to effect a competition reaction and simultaneously free labelled antigens (or labelled antibodies) which are not bound to an immobilized antibody are washed out. Thereafter, the substrate solution supplied from the substrate solution reservoir and a labelled enzyme are reacted to form a color or occur a signal (fluorescence, light emission, etc.) which is detectable. The labelled antigen (or the labelled antibody) does not contact the substrate solution after being immobilized to the detecting zone until developing the substrate solution in the substrate solution reservoir, so that no enzyme inhibitor is required for inhibiting a reaction of the labelled enzyme during moving the labelled antigen (or the labelled antibody). It is sufficient to provide a labelling reagent zone at a position downstream of the washing solution reservoir; the position of the specimen dotting zone is not particularly limited.

When the specimen to be detected is an antibody, an immunoassay device can be constituted in the same manner by adding an immobilized antigen in place of the immobilized antibody to the above detecting zone.

Moreover, in another preferred embodiment of the present invention, an immunoassay device for assaying an antigen to be tested in a sample solution is provided by the following constituents:
(a) a matrix which can transfer a solution by capillarity,
(b) a specimen dotting zone provided in the above matrix, to which a sample solution containing an antigen to be tested is dotted,
(c) a labelling reagent zone containing an antigen or antibody which is labelled by a labelling enzyme and provided in the matrix at a position separate from the specimen dotting zone,
(d) a detecting zone containing an immobilized antibody and provided in the matrix at a position downstream, with respect to the direction of solution transfer, of the specimen dotting zone and the labelling reagent zone,
(e) a substrate solution reservoir containing a substrate of the labelling enzyme and provided in the matrix at a position upstream, with respect to the direction of solution transfer, of the specimen dotting zone and the labelling reagent zone, and
(f) a washing solution reservoir provided in the matrix at a position between the labelling reagent zone and the specimen dotting zone.

In the above embodiment, a washing solution reservoir is provided between a labelling reagent zone and a specimen dotting zone. By supplying a washing solution in the washing solution reservoir to a matrix prior to development and movement of a substrate solution from a substrate solution reservoir, either of an antigen to be tested or a labelled antigen (or a labelled antibody) is transferred by a solution and moved to a detecting zone to bind to an immobilized antibody. Free antigens to be tested or labelled antigens (or the labelled antibody) which are not bound to the immobilized antibody are washed out by the washing solution. Thereafter, the remaining labelled antigen (or the labelled antibody) or the antigen to be tested reaches to the detecting zone with the substrate solution supplied from the substrate solution reservoir. Unreacted reagent (free antigen or antibody) has already been removed, and an immuno reaction proceeds stepwisely so that a diphasic property due to a prozone effect does not appear even if antigens to be tested exist excessively.

When a specimen to be detected is an antibody, an immunoassay device can be constituted in the same manner by adding an immobilized antigen in place of the immobilized antibody to the above detecting zone. Also, in this case, an enzyme-labelled second antibody may be impregnated in the labelling reagent zone.

Furthermore, in the most preferred embodiment of the present invention, an immunoassay device for assaying an antigen to be tested in a sample solution is provided by the following constituents:
(a) a matrix which can transfer a solution by capillarity,
(b) a specimen dotting zone provided in the above matrix, to which a sample solution containing an antigen to be tested is dotted,
(c) a labelling reagent zone containing an antigen or antibody which is labelled by a labelling enzyme and provided in the matrix at a position upstream, with respect to the direction of solution transfer, of the specimen dotting zone,
(d) a detecting zone containing an immobilized antibody and provided in the matrix at a position downstream, with respect to the direction of solution transfer, of the specimen dotting zone,
(e) a substrate solution reservoir containing a substrate of the labelling enzyme and provided in the matrix at a position upstream, with respect to the direction of solution transfer, of the labelling reagent zone,
(f) a first washing solution reservoir provided in the matrix at a position downstream, with respect to the direction of solution transfer, of the labelling reagent zone and upstream of the specimen dotting zone, and
(g) a second washing solution reservoir provided on the matrix at a position upstream, with respect to the direction of solution transfer, of the labelling reagent zone and downstream of the substrate solution reservoir.

In the above embodiment, a first washing solution is firstly supplied from a first washing solution reservoir to bind an antigen to be tested to an immobilized antibody in a detecting zone. Next, a second washing solution from a second washing reservoir is supplied to move a labelled antigen or a labelled antibody to the detecting zone. When a labelled antigen is used, the labelled antigen binds to the immobilized antibody which is not bound to the antigen to be tested. When a labelled antibody is used, the labelled antibody binds to the antigen to be detected which is bound to the immobilized antibody in the form of sandwiching the antigen to be detected. The labelled enzyme of the labelled antigen (or the labelled antibody) directly (or indirectly) bound to the immobilized antibody as mentioned above finally forms a color by reacting with a substrate solution supplied from a substrate solution reservoir.

Also, in this embodiment, since respective reagents are supplied and reacted stepwisely, so-called prozone phenomenon does not occur. Further, since an unreacted labelled antigen (or a labelled antibody) has been removed by the washing solution supplied from the second washing solution reservoir, it is not necessary to use an enzyme inhibitor in order to inhibit an enzyme reaction due to a free labelled antigen (or a labelled antibody).

In the above respective embodiments, an enzyme labelling reagent zone, a specimen dotting zone and a detecting zone have been previously provided to a matrix, depending on necessity, an area to which respective reagents or a specimen is dotted when using may be used as respective zones. Also, a substrate solution may be supplied from an end of a matrix when using without providing a substrate solution reservoir.

### BRIEF DESCRIPTION OF THE DRAWINGS

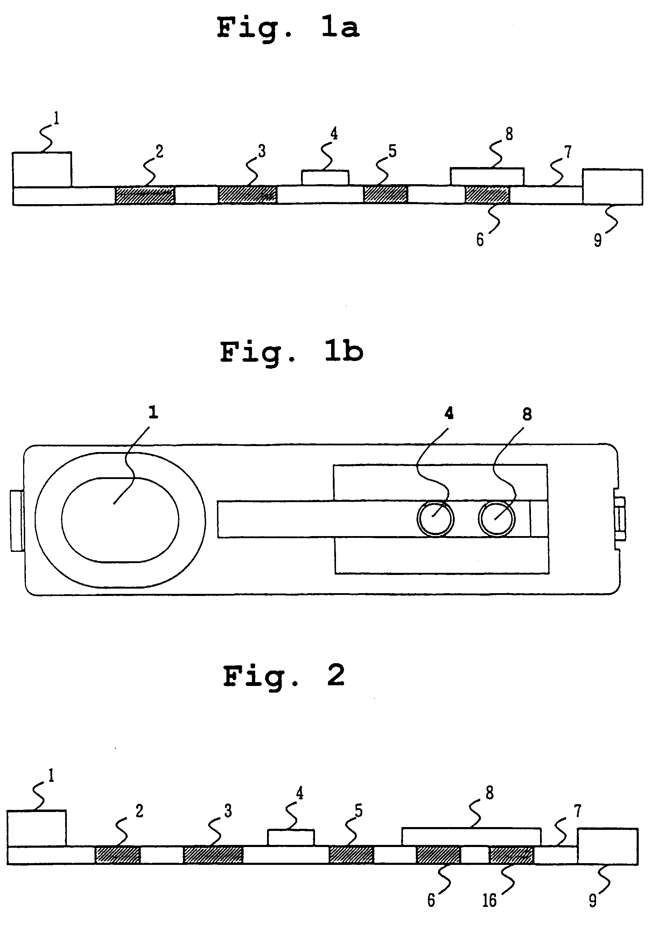

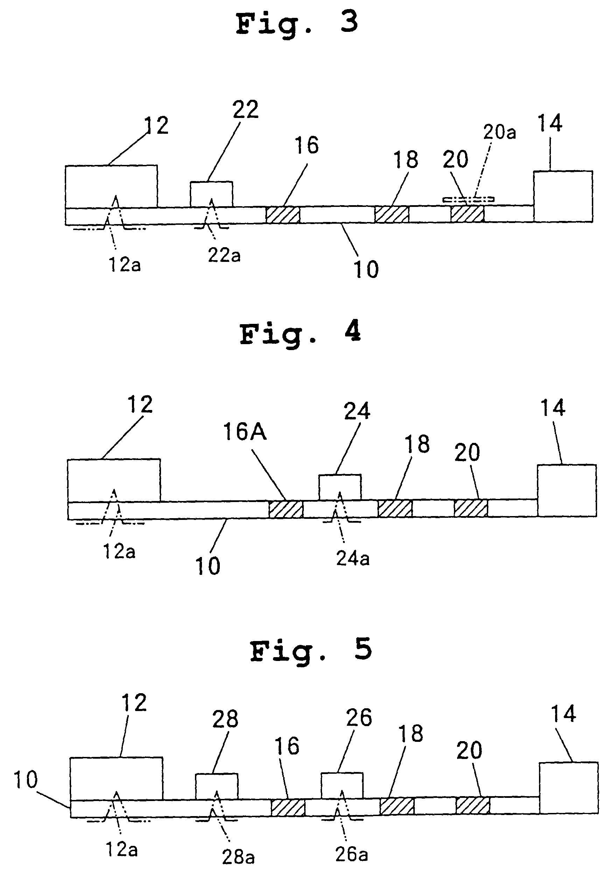

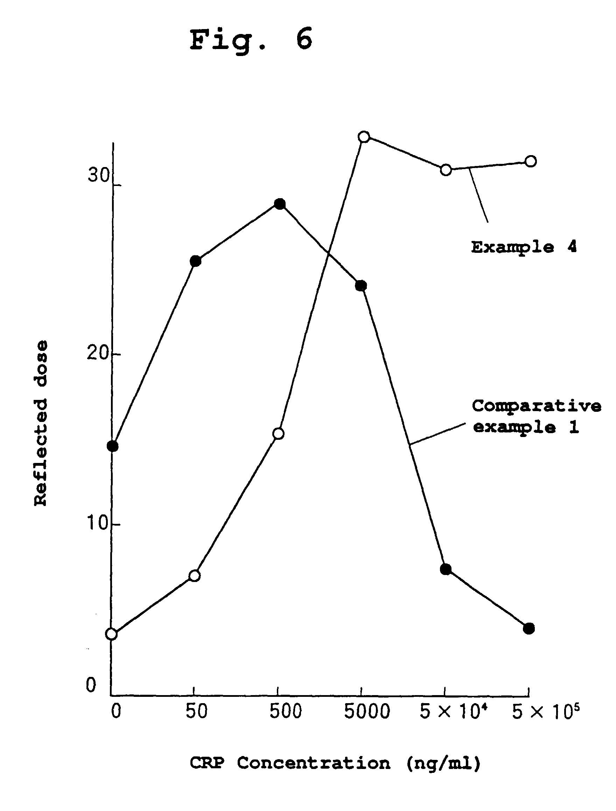

Fig. 1a is a sectional view showing a constitutional example of a test strip for simple and easy immunoassay; Fig. 1b is a plan view showing a constitutional example of a test strip; Fig. 2 is a sectional view showing a constitutional example of a test strip for simple and easy immunoassay, which is effecting to measurement of two items; Fig. 3 is a conceptional view showing a first embodiment of an immunoassay device of the invention; Fig. 4 is a conceptional view showing a second embodiment of an immunoassay device of the invention; Fig. 5 is a conceptional view showing a third embodiment of an immunoassay device of the invention; Fig. 6 is a calibration curve showing experimental results of Example 4 and Comparative example 1; Fig. 7 is a calibration curve showing experimental results of Example 5; and Fig. 8 is a conceptional view of an immunoassay device of a prior art.

### DESCRIPTION OF THE PREFERRED EMBODIMENTS

According to the present invention, after dotting a specimen, a developing solution containing a substrate in a test strip of the present invention is firstly broken to impregnate a developing solution into a developing material whereby measurement is initiated. The substrate contained in the developing solution includes, needless to say, those for an enzyme to be used in the present invention such as alkaliphosphatase, peroxidase and β-galactosidase, and also includes, for example, 3-(2'-spiroadamantane)-4-methoxy-4-(3"-phosphoryloxy)phenyl-1,2-dioxetane (AMPPD, for alkaliphosphatase), luminol-H₂O₂ (for peroxidase), 3-(2'-spiroadamantane)-4-methoxy-4-(3"-β-D-galactopyranosyl)phenyl-1,2-dioxetane (AMGPD, for β-galactosidase). These substrates can be used by adding to various kinds of buffers to be used as a developing solution. As a buffer, there may be mentioned, for example, acetate buffer, borate buffer, Tris-HCl buffer and diethanolamine buffer.

Then, the developing solution continue to impregnate into the developing material to reach to one of a reagent zone. The reagent zone is a reagent zone which restrains light emission and to the zone were added, for example, a reaction inhibitor to the above enzymes acting on a substrate and a light emission reaction inhibitor of a light emission substrate. As an enzyme reaction inhibitor, to the alkaliphosphatase, there may be mentioned, for example, an organic phosphoric acid such as phenylphosphoric acid, an inorganic phosphoric acid such as ortho-phosphoric acid, and a chelate compound such as EDTA (ethylenediaminetatraacetate) and EGTA (ethylene glycol (2-aminoethyl ether)-N,N,N',N'-tetraacetic acid); to the peroxidase, there may be mentioned, for exam le, ascorbic acid, sodium azide and catalase; and to the β-D-galactosidase, there may be mentioned, for example, a galactopyranoside derivative such as phenyl-s-β-galactopyranoside and phenyl-o-β-galactopyranoside.

As the light emission reaction inhibitor, there may be mentioned, for example, various kinds of buffers such as a phosphate buffer having a specific pH and Tris-HCl buffer, and various kinds of dyes. The dye may include a dye having a same absorption wavelength as a light emission wavelength such as Reactive Yellow having an absorption wavelength of about 400 nm, Reactive Red having an absorption wavelength of about 500 nm and Reactive Blue having an absorption wavelength of about 550 nm.

Further, the developing solution containing respective reagents permeates and reaches to an enzyme-labelling reagent zone. In the zone, a reaction of the substrate in the developing solution and an enzyme of an enzyme-labelling reagent to be present in this zone is inhibited by the above inhibitor. The enzyme can be used by selecting from the aforesaid enzymes, and it can be used as an enzyme-labelling antibody by binding to an antibody corresponding to an object to be measured. The object to be measured of the present invention may be mentioned, for example, a chemical such as theophylline, phenytoin and varproic acid; a low molecular weight hormone such as thyroxine, estrogen and elastoradiol; a cancer marker such as CEA (carcinoembryonic antigen), AFP (α-fetoprotein) and hemoglobin in feces; a virus such as human immunodeficiency virus (HIV), human adult T-cell leukemia virus (HTLV-I) and hepatitis B virus (HBV); a high molecular weight hormone such as thyroid-stimulating hormone (TSH) and insulin; a cytokine such as IL-1 (interleukin-1), IL-2 and IL-6; various kinds of gloss factors such as EGF (epitheliocyte growth factor) and PDGF (platelet derived growth factor); and further suitable DNA and RNA of the above viruses; an antigen such as a protein relating to an inflammation as CRP (C-reactive protein) and an antibody to the antigen. The antibody in the enzyme-labelling antibody may be a monoclonal antibody or a polyclonal antibody to the above object to be measured, and also it may be a decomposed product having an antigen-recognizing part of these antibodies such as Fab, Fab' and F(ab')₂. Various antibodies can be obtained by combining the known methods (for example, see "Monoclonal Antibody and Cancer", published by K.K. Science Forum (1985)). Bound product of an enzyme and an antibody can be prepared by utilizing a known method for forming a covalent bond or a non-covalent bond (for example, see "Protein, Nucleic Acid and Enzyme", special edition, No. 31, pp. 37 to 44 (1987)).

Further, the developing solution reaches to a specimen dotting zone. In this zone, a reaction of the above enzyme-labelled antibody and an object to be measured occur when the object to be measured is present in a specimen, whereby an immuno complex of the object to be measured and the enzyme-labelled antibody is formed.

Also, the developing solution containing each reagent and the immuno complex reaches to a zone which starts light emission. In this zone, as a light emission initiating reagent, for example, an acetate buffer, a borate buffer, a Tris-HCl buffer and a diethanolamine buffer which are a buffer capable of shifting a pH of the above buffer containing the substrate to an optimum pH which develops enzyme activity may be present.

Then, the developing solution reaches to a detecting zone which is adjacent to the initiating zone. In the detecting zone, an antigen or an antibody can be used by binding them to a solid phase. In the detecting zone, the antigen or the antibody immobilized to the solid phase and the above immuno complex of the object to be measured and the enzyme-labelled antibody are reacted to fix the enzyme on the solid phase. The substrate reacts with the enzyme to cause light emission. By measuring an emitted amount, an amount of the object to be measured can be determined. These antigen and antibody can be determined depending on the object to be measured in a specimen. As the antibody, there may be used the above monoclonal antibody, polyclonal antibody or an antibody decomposed material having a part recognizing an antigen.

The solid phase to bind the antigen or antibody may include, for example, a polystyrene latex, various kinds of animal blood cells and gelatin particles (see Japanese Patent Publication No. 29223/1988). Also, it may be used by directly fixing to the detecting zone of the developing material.

A method for binding to the solid phase, a physical adsorption method or a chemical binding method can be used. The physical adsorption method is carried out by reacting the above solid phase and an antigen or an antibody in a suitable buffer. As the buffer to be used in the reaction, there may be mentioned, for example, a phosphate buffer, a Tris-HCl buffer and a carbonate buffer. The reaction readily proceeds by mixing both of the components at 4 °C to 37 °C, preferably at room temperature to obtain a desired product. Also, as the chemical binding method, a carbodiimide method used in the so-called peptide binding method can be used. Further, as the other chemical binding method, a method in which a reaction is carried out in the presence of a divalent cross-linking agent such as glutaraldehyde and cyanuric chloride (see "Peptide Synthesis Method", published by Maruzen K.K. (published in 1975), "Enzyme Immunoassay Method", published by Kyoritsu Shuppan K.K., and "Protein, Nucleic acid and Enzyme" special edition, No. 31 (1987)). When no functional group is present at the solid phase, after processing the surface of the solid phase with a reagent having a functional group, the above chemical binding method can be applied to.

The developing material having capillarity of the present invention may be any material which can transfer or move the developing solution according to the above phenomenon. As such a developing material, there may be mentioned, for example, a water-absorptive filter paper containing cellulose and a filter paper containing a glass fiber. Also, at the opposite end to the developing solution reservoir of the test strip, an absorption pad which absorbs the developing solution may be added to, if necessary. This absorption pad is a device for absorbing the developing solution when the developing solution is developed and reached to the end whereby continuing development easily. As the absorption pad, it may be used by selecting from various materials having water-absorption property.

The test strip for immunoassay of the present invention can be prepared, for example, according to the method as shown below. At an appropriate zone of a filter paper which is a developing material, the above inhibitor, the enzyme-labelled antibody and the antibody or antigen-bound solid phase are separately dropped in an amount of 10 µl to 200 µl, preferably 20 µl to 70 µl and dried. As the drying method, there may be used a method in which a freeze-drying apparatus is used, air drying method or reduced-pressure drying method. Further, as a method of using the strip simply and easily, the dried test strip can be used by setting it to an apparatus in which the developing solution containing a substrate is previously sealed with e.g. an aluminum laminate so as to become a bag state and provided to one end of a plastic case, and sealing thereon with a plastic case to which a rectangular or circular hole is previously provided as a specimen dotting zone.

To the specimen dotting zone of the test strip thus prepared was dotted 10 µl to 300 µl, preferably 30 µl to 70 µl of a specimen, and a bag portion of the developing solution containing a substrate solution is broken by, for example, pushing with fingers to permeate the solution to a filter paper of the test strip. After 1 min to 20 min, preferably after 2 min to 10 min, exposure is carried out by exposing a light-sensitive film or a printing paper attached to the detecting zone to read a light emission dose. Further, in order to obtain an accurate light emission dose, a dose can be directly counted at the detecting zone by a photodiode or a photomultiplier. Also, the exposed film or printing paper can be judged with eyes or the exposed surface can be read by a differential colorimeter.

A specimen containing an object to be measured of the present invention is not particularly limited, and may include, for example, blood plasma, serum, urine, an extract of blood cells, whole blood and an extract of whole blood.

Next, the test strip of the present invention will be explained in detail by referring to the drawings. Fig. 1 is one example showing a constitution of the test strip of the present invention. As a test strip for practicing the present invention, it possesses a developing solution layer 1 containing a substrate, a developing material 7 and an absorption pad 9. In the developing material 7, a reagent zone 2 which inhibits light emission, a reagent zone 5 which initiates light emission, an enzyme-labelling reagent zone 3, a detecting zone 6 and a specimen dotting zone 4 may be provided. Onto the detecting zone 6, a device 8 which detects emitted light can be provided. For example, the aforesaid light-sensitive film or printing paper may be used.

Fig. 2 is an example showing a constitution for measuring, for example, two items of objects to be measured, by using a test strip of the present invention. In addition to the respective constitution shown in Fig. 1, in the enzyme-labelled antibody reagent zone 3, two kinds of enzyme-labelled antibodies to the objects to be measured may be contained. Also, for detection, two places of detecting zones 6 and 16 may be provided to.

In the following, the immunoassay device of the present invention will be explained in detail.

### Whole constitution of the first embodiment

In Fig. 3, a sectional view of the first embodiment of the immunoassay device of the present invention is shown. In this figure, the reference numeral 10 is a matrix which comprises a water-absorbable material capable of transferring a developing solution by capillarity. A preferred material includes a filter paper which may be formed by cellulose or a glass fiber.

The matrix 10 is formed in a shape of a strip, and a substrate solution reservoir 12 is provided at one end to the longer direction thereof and a water-absorption promoting member (absorption pad) 14 is provided adjacent to a filter paper at the other end thereof. Between the substrate solution reservoir 12 and the absorption pad 14, a labelling reagent zone 16, a specimen dotting zone 18 and a detecting zone 20 are successively provided so as to cross the longer direction. A washing solution reservoir 22 is provided at a position between the substrate solution reservoir 12 and the labelling reagent zone 16.

### Specimen dotting zone

The specimen dotting zone 18 uses part or area of the matrix 10 as it were. As a specimen to be dotted to the specimen dotting zone 18, there may be mentioned a liquid component such as blood plasma, serum, urine, an extract of blood cells, whole blood and an extract of whole blood. In case where a non-liquid component such as blood cells is contained in a specimen, the device may be so constituted as to permeate the liquid component alone into the specimen dotting zone when using by providing a filter layer on the specimen dotting zone.

As an object to be measured in the specimen, the following antigen and antibody may be mentioned.
1) Chemicals: theophylline, phenytoin and varproic acid,
2) Low molecular weight hormone: thyroxine, estrogen and elastoradiol,
3) Cancer marker: carcinoembryonic antigen (CEA), α-feto-protein (AFP) and hemoglobin in feces,
4) Virus: human immunodeficiency virus (HIV), human adult T-cell leukemia virus (HTLV-I) and hepatitis A, B or C virus,
5) High molecular weight hormone: thyroid-stimulating hormone (TSH) and insulin,
6) Cytokine: interleukin-1 (IL-1), IL-2 and IL-6,
7) Cell growth factor: epitheliocyte growth factor (ECGF) and platelet derived growth factor (PDGF),
8) Virus DNA and RNA,
9) Antigen relating to inflammation: C-reactive protein (CRP),
10) Antibody to the above antigen and fragment having an antigen-recognizing part of these antibodies such as Fab, Fab' and F(ab')₂.

### Labelling reagent zone

In the labelling reagent zone 16, an enzyme-labelling reagent is impregnated. When an object to be measured in a specimen is an antigen, an enzyme-labelled antigen or an enzyme-labelled antibody is impregnated as an enzyme-labelling reagent. When an object to be measured in a specimen is an antibody, an enzyme-labelled antigen, an enzyme-labelled antibody or an enzyme-labelled second antibody is impregnated

When an antibody is used, the antibody may be an antibody obtained by a usual method, but if a monoclonal antibody is used, sensitivity is more improved. Also, the antibody may be a fragment such as F(ab')₂, Fab' or Fab.

A labelling enzyme may be any one which forms a signal (coloring, fluorescence or light emission) which is detectable directly or indirectly by reacting with a substrate solution supplied from a substrate solution reservoir 12. Examples of these labelling enzymes may include alkali phosphatase, peroxidase and β-D-galactosidase.

A method for labelling an enzyme can be utilized a known method described in, for example, an enzyme immunoassay method ("Protein·Nucleic Acid·Enzyme", special edition, No. 31 (1987), edited by Eiji Ishikawa et al., Kyoritu Shuppan).

### Detecting zone

In the detecting zone 20, an immobilized antibody is contained when an object to be measured is an antigen and an immobilized antigen is contained when an object to be measured is an antibody. Immobilization of these antibody and antigen may be carried out by directly binding to a matrix with a covalent bond or by binding to an insoluble carrier and contained it in a matrix. As such an insoluble carrier, there may be mentioned, for example, a polystyrene latex, red blood cells of various kinds of animals, gelatin particles (as disclosed in Japanese Patent Publication No. 29223/1988) and a glass fiber. The antibody or antigen to be contained in the detecting zone is to be constituted so as not to move from the detecting zone by a washing solution supplied from a washing solution reservoir 22 or a substrate solution supplied from a substrate solution reservoir 12.

### Substrate solution reservoir

The substrate solution reservoir 12 contains a substrate solution containing a reactive substrate of a labelling enzyme to a labelled antibody or labelled antigen. The substrate solution reservoir 12 may have a structure in which the substrate solution can be supplied to a matrix 10 when using. For example, by constituting a bag comprising a material which is capable of splitting (e.g. an aluminum foil, a polyethylene film), and splitting the bag when using, a washing solution therein can be supplied to the matrix 10. A volume of the substrate solution reservoir 12 may be a size which can sufficiently supply the substrate solution to a detecting zone 20 and can secure an enzyme reaction in the detecting zone sufficiently. This volume is determined in view of a volume of a washing solution reservoir 22.

A reactive substrate in the substrate solution may be any material which forms a directly or indirectly detectable signal (coloring, fluorescence or light emission) by the reaction with a labelling enzyme, and a co-enzyme necessary for the enzyme reaction is also included. In order to optically detect a product formed by the reaction with the labelling enzyme, a known detecting reagent composition may be also contained in combination.

The reactive substrate can be selected a suitable one from a known reagent depending on the kind of the labelling enzyme. For example, the following can be mentioned.
a) Coloring substrate:
   3,3',5,5'-tetramethylbenzidine (TMB), 2,2'-azinodi(3-ethylbenzthiazolin-6'-sulfonic acid (ABTS) and 3,3'-diaminobenzidine (DAB) as a substrate for peroxidase,
   5-bromo-4-chloro-3-indolylphosphoric acid (BCIP) as a substrate for alkaliphosphatase.
b) Fluorescent substrate:
   4-methylumbelliferyl·phosphate (4MUP) as a substrate for alkaliphosphatase,
   4-methylumbelliferyl·β-D-galactoside (4MUG) as a substrate for β-D-galactosidase.
c) Light emission substrate:
   1,2-dioxetane derivative such as disodium 3-(2'-spiroadamantan)-4-methoxy-4-(3"-phosphoryloxy)phenyl-1,2-dioxetane (AMPPD) and disodium 3-(4-methoxyspiro[1,2-dioxetane-3,2'-(5'-chloro)tricyclo[3.3.1.1^{3,7}]decan]-4-yl)phenyl phosphate dioxetane salt (Cℓ-AMPPD) as a substrate for alkaliphosphatase, and
   3-(2'-spiroadamantan)-4-methoxy-4-(3"-β-D-galactopyranosyl)phenyl-1,2-dioxetane (AMGPD) and disodium 3-(4-methoxyspiro[1,2-dioxetane-3,2'-(5'-chloro)tricyclo[3,3,1,1^{3,7}]decan]-4-yl)phenyl β-D-galastopyranose (Cℓ-AMPGD) as a substrate for β-D-galactosidase;
   ISO-luminol or luminol and hydrogen peroxide as a substrate for peroxidase.

To the substrate solution, an activator of an enzyme, a co-enzyme, a surfactant and a pH buffer (e.g. a buffer having a pH of 5.5 or so such as a citrate buffer) may be optionally added. As a preferred buffer, there may be mentioned acetate buffer, borate buffer, Tris-HCl buffer and diethanolamine buffer.

### Washing solution reservoir

In the washing solution reservoir 22, a washing solution is contained for washing out a labelled antigen (or labelled antibody) from a labelling reagent zone 16 and an antigen to be tested (or antibody to be tested) of an object to be measured from a specimen dotting zone 18, and for transferring them to a detecting zone 20. The washing solution reservoir 22 may have a structure in which a substrate solution is to be supplied to a matrix 10 when using. For example, by constituting a bag comprising a material which is capable of splitting (e.g. an aluminum foil, a polyethylene film), and splitting the bag when using, a washing solution therein can be supplied to the matrix 10. A volume thereof may be a size which can sufficiently transfer the labelled antigen (or labelled antibody) from the labelling reagent zone 16, and an antigen to be tested (or antibody to be tested) of an object to be measured from a specimen dotting zone 18 to the detecting zone 20, and can wash out free antigen to be tested and the labelled antigen (or labelled antibody) at the detecting zone 20 sufficiently.

The washing solution may be a solution which does not participate any bad effect to immuno reaction, and there may be used, for example, phosphate bufferred physiological saline solution (PBS) and acetate buffered physiological saline solution. If necessary, a surfactant is used, it is effective for removing and washing unreacted antibody or antigen and unnecessary protein in a specimen. Preferred surfactant may include Tween 20 (trade name), Triton X-100 (trade name), polyvinyl pyrrolidone (PVP) and polyvinyl alcohol (PVA).

### Water-absorption promoting member

The water-absorption promoting member 14 is used in order to promote development and movement of a washing solution supplied from a washing solution reservoir 22 and a substrate solution supplied from a substrate solution reservoir 12 thereafter in a matrix 10 smoothly. In order to secure one direction movement (a direction from the left side to the right side in Fig. 3) of a liquid component in the matrix 10, the water-absorption promoting member 14 preferably comprises a material having a higher water-absorptive property or a larger water-retention capacity than that of the matrix 10. For example, sponge, or filter papers having higher water retention characteristics than the matrix 10 being laminated can be used as the water-absorption promoting member 14.

It is not necessary to provide such a water-absorption promoting member 14 if the matrix 10 can be made sufficiently long and water-absorption ability at the downstream side of development than the detecting zone 20 can be secured. However, in order to shorten a development time of liquid components and to minimize a device as a whole, it is preferred to provide such a water-absorption promoting member.

### Method of use

In order to simplify an explanation, explanation is made in the case where an object to be measured is an antigen and a labelling reagent contained in a labelling reagent zone 16 is an enzyme-labelled antibody. In this case, an immobilized antibody is contained in a detecting zone 20.

After dotting a specimen to a specimen dotting zone 18, a washing solution reservoir 22 is broken to supply a washing solution to a matrix 10. When a projected member 22a which penetrates the matrix 10 is provided at the washing solution reservoir 22 beneath, it breaks the washing solution reservoir 22 by pressing whereby the washing solution can be easily supplied to the matrix 10.

The washing solution supplied to the matrix 10 develops to the right direction of Fig. 3 by capillarity, and an enzyme-labelled antibody and an antigen to be measured are successively washed out by the development top end thereof from the labelling reagent zone 16 and the specimen dotting zone 18, respectively, to reach to the detecting zone 20. At the detecting zone 20, the immobilized antibody and the antigen to be measured are reacted, and the antigen to be measured is immobilized in a shape that it is sandwiched between the immobilized antibody and the enzyme-labelled antibody (so-called one step sandwich method). Free labelled-antigen and the enzyme-labelled antibody which are not bound to the immobilized antibody are washed out to the direction from the detecting zone 20 to the water-absorption promoting member 14 by the washing solution which is still continuously supplied to.

After completion of development of the washing solution, then a substrate solution reservoir 12 is broken by pressing to a projected member 12a which penetrates the matrix 10 to supply a substrate solution to the matrix 10. The substrate solution develops in the matrix 10 and reaches to the detecting zone 20. A substrate in the substrate solution reacts with a labelling enzyme of the labelled antibody bound to the immobilized antibody to form an optically detectable signal such as a dye. At this time, since an unreacted free labelled antibody has already been removed by the washing solution, it does not become a noise source.

An amount of the formed dye can be measured by a reflective light measurement system or a color difference meter and then an amount of the antigen to be measured in the specimen can be obtained according to the principal of the colorimetric method using a calibration curve previously prepared. Also, by confirming the presence or absence of a formed dye with eyes, the presence or absence of the antigen to be measured can be qualitatively determined. When color charts corresponding to respective antigen concentration is prepared, semi-quantitative measurement can be done even when judgements with eyes are effected.

When a fluorescent substrate is used as an enzyme substrate, it is measured by a fluorophotometer. Also, when a light emission substrate is used, an emitted dose can be countered by a photocounter, but by previously providing an instant film 20a shown with a dashed line in Fig. 3 over the detecting zone 20, an exposed dose of the film 20a may be measured after assay.

The above explanation relates to a method to which a sandwich method is applied, but if an enzyme-labelled antigen is used in place of the enzyme-labelled antibody, competitive reaction type assay can be also done. In this case, an amount of the dye formed at the detecting zone 20 becomes in inverse proportion to an amount of the antigen to be measured.

When a combination of an object to be measured, a labelling reagent in a labelling reagent zone 16 and an immobilizing reagent in a detecting zone 20 is summarized as shown in Table 1 below.

**Table 1**

| Object to be measured | Labelling reagent zone | Detecting zone | Reaction system |
|---|---|---|---|
| 1) Antigen | Enzyme-labelled antigen | Immobilized antibody | Competitive type |
| 2) Antigen | Enzyme-labelled antibody | Immobilized antibody | Sandwich type |
| 3) Antibody | Enzyme-labelled antibody | Immobilized antigen | Competitive type |
| 4) Antibody | Enzyme-labelled antigen | Immobilized antibody | Sandwich type |
| 5) Antibody | Enzyme-labelled second antibody | Immobilized antigen | Sandwich type |
| 6) Antibody | Enzyme-labelled antigen | Immobilized second antibody | Sandwich type |

The second antibody used in Table 1 is an antibody to an antibody which is an object to be measured, and depending on Ig class of the antibody which is an object to be measured, anti-IgG, anti-IgM or anti-IgE is used. This is the same also in a second antibody mentioned in the following other embodiments.

In Fig. 3, whereas the labelling reagent zone 16 is present upstream than the specimen dotting zone 18, this configuration may be reversed. It may be sufficient that at least the labelling reagent zone 16 is positioned at downstream the washing solution reservoir 22.

### Second embodiment of the immunoassay device

In the second embodiment shown in Fig. 4, a washing solution reservoir 24 is provided at a downstream side of a labelling reagent zone 16 and at an upstream side of a specimen dotting zone 18. Reference numerals in Fig. 4 are the same meanings as those in Fig. 3 otherwise specifically mentioned. Constitution of the washing solution reservoir 24 in Fig. 4 is the same as that of the washing solution reservoir 22 in Fig. 3 and only the position provided is different. Provided that, in a labelling reagent zone 16A, an enzyme inhibitor which inhibits a reaction of the enzyme is also contained in addition to an enzyme-labelling reagent, which is different from that of the first embodiment in Fig. 3.

A method of use where an object to be measured is an antigen and a labelling reagent contained in the labelling reagent zone 16A is an enzyme labelled antibody is explained. In this case, an immobilized antibody is contained in a detecting zone 20.

After dotting a sample solution containing an antigen to be measured to the specimen dotting zone 18, the washing solution reservoir 24 is broken by pressing to a projected member 24a provided beneath thereof to supply a washing solution to a matrix 10. The washing solution which develops and moves to right direction in Fig. 4 by capillarity washes out at the development top end thereof the antigen to be measured from the specimen dotting zone 18 to transfer to the detecting zone, and the antigen to be measured is reacted with the immobilized antibody in the detecting zone 20. When free antigens to be measured which are not bound to the immobilzed antibody due to excess amount of the antigen to be measured are present, they can be washed out and removed from the detecting zone 20 by the washing solution which is still continuously supplied.

After completion of development of the washing solution, then a substrate solution reservoir 12 is broken by pressing to a projected member 12a which penetrates the matrix 10 to supply a substrate solution to the matrix 10. The substrate solution develops in the matrix 10 and washes the enzyme-labelled antibody out of the labelling reagent zone 16A to move to the detecting zone 20. The enzyme-labelled antibody binds at the detecting zone 20 to the antigen to be measured which is bound to the immobilized antibody, and immobilized in the shape that the immobilized antibody is sandwiched by the antigen to be measured. That is, it becomes a two step sandwich type reaction system. Unreacted labelled-antibody and the enzyme inhibitor are washed out and removed by the substrate solution which is still continuously supplied to. The labelled-enzyme immobilized by removal of the enzyme inhibitor reacts with the substrate to form a detectable signal such as a dye.

In this embodiment, a binding reaction of an antigen, an immobilized antibody and an enzyme-labelled antibody can be effected stepwisely and free antigens which did not bind to the immobilized antibody at the first step reaction can be removed, so that prozone phenomenon does not occur even when en excessive amount of the antigen to be measured is present. Thus, assay at high concentration range can be done.

In the above explanation, two step sandwich method is applied to, but an enzyme-labelled antigen may be used in place of the enzyme-labelled antibody. In this case, the larger an amount of the antigen to be measured becomes, the less an amount of the enzyme-labelled antigen which can be bound to the immobilized antibody is. Thus, an assay at a higher concentration range can be realized.

The above explanation relates to a method to which a two step sandwich method is applied, but an enzyme-labelled antigen can be used in place of the enzyme-labelled antibody. In this case, the larger an amount of the antigen to be measured becomes, the less an amount of the enzyme-labelled antigen which can be bound to the immobilized antibody is.

When a combination of an object to be measured, a labelling reagent in a labelling reagent zone 16A and an immobilizing reagent in a detecting zone 20 is summarized as shown in Table 2 below.

**Table 2**

| Object to be measured | Labelling reagent zone | Detecting zone | Reaction system |
|---|---|---|---|
| 1) Antigen | Enzyme-labelled antigen | Immobilized antibody | --- |
| 2) Antigen | Enzyme-labelled antibody | Immobilized antibody | Sandwich type |
| 3) Antibody | Enzyme-labelled antibody | Immobilized antigen | --- |
| 4) Antibody | Enzyme-labelled antigen | Immobilized antibody | Sandwich type |
| 5) Antibody | Enzyme-labelled second antibody | Immobilized antigen | Sandwich type |
| 6) Antibody | Enzyme-labelled antigen | Immobilized second antibody | Capturing type |

### Third embodiment of the immunoassay device

In the third embodiment shown in Fig. 5, two washing solution reservoirs are provided to. That is, as in the first embodiment shown in in Fig. 3, a substrate solution reservoir 12 is provided at the most upstream side of a matrix 10 and an absorption promoting member 14 is provided at the lowest downstream side of the same. Between the substrate solution reservoir 12 and the the absorption promoting member 14 of the matrix 10, a labelling reagent zone 16, a specimen dotting zone 18 and a detecting zone 20 are successively provided from the upstream side. These constitutions are completely the same as those of the first embodiment. Further, a first washing solution reservoir 26 is provided between the labelling reagent zone 16 and the specimen dotting zone 18, and a second washing solution reservoir 28 is provided between the substrate solution reservoir 12 and the labelling reagent zone 16. The constitutions of these two washing solution reservoirs 26 and 28 are the same as the washing solution reservoir 22 in the first embodiment. It is also the same that projecting members 26a and 28a are provided at the beneath of respective reservoirs in order to break the reservoir.

A method of use where an object to be measured is an antigen and a labelling reagent contained in the labelling reagent zone 16 is an enzyme labelled antibody is explained below. In this case, an immobilized antibody is contained in the detecting zone 20.

After dotting a sample solution containing an antigen to be measured to the specimen dotting zone 18, the first washing solution reservoir 26 is broken to supply a washing solution to the matrix 10. Thereby the antigen to be measured in the specimen dotting zone 18 is washed out to be transferred to the detecting zone 20 so that it is bound to the immobilized antibody. Excessive antigen to be measured is washed out and removed from the detecting zone 20 by the washing solution which is still continuously supplied.

After completion of development of the first washing solution, when the second washing solution reservoir 28 is broken to supply a second washing solution to the matrix 10, a labelled antibody in the labelling reagent zone 16 is washed out to be transferred to the detecting zone 20. The enzyme-labelled antibody binds to the antigen to be measured which is bound to the immobilized antibody in the detecting zone 20 whereby immobilizing in the form of sandwiching the antigen to be detected with the immobilized antibody. That is, it becomes a two step sandwich type reaction system. Unreacted labelled-antibody is washed out and removed from the detecting zone 20 by the second washing solution which is still continuously supplied to. Finally, the substrate solution reservoir 12 is broken and the substrate solution is supplied to the matrix 10, so that the substrate reaches to the detecting zone 20 and reacts with the labelled-enzyme of the labelled antibody which is immobilized at the detecting zone 20. As the results, a detectable signal such as a dye corresponding to an amount of the antigen to be measured is occurred.

In this embodiment, a binding reaction of an antigen, an immobilized antibody and an enzyme-labelled antibody can be effected stepwisely, so that there are effects that prozone phenomenon can be prevented and a concentration range which can be measured can be broadened. Also, unreacted labelled antibody can be removed by the washing solution supplied from the second washing solution reservoir 28 so that it does not become a noise source.

When a combination of an object to be measured, a labelling reagent in a labelling reagent zone 16 and an immobilizing reagent in a detecting zone 20 is summarized as shown in Table 3 below.

**Table 3**

| Object to be measured | Labelling reagent zone | Detecting zone | Reaction system |
|---|---|---|---|
| 1) Antigen | Enzyme-labelled antigen | Immobilized antibody | --- |
| 2) Antigen | Enzyme-labelled antibody | Immobilized antibody | Sandwich type |
| 3) Antibody | Enzyme-labelled antigen | Immobilized antigen | --- |
| 4) Antibody | Enzyme-labelled second antibody | Immobilized antigen | Sandwich type |
| 5) Antibody | Enzyme-labelled antigen | Immobilized second antibody | Capturing type |

In No. 4) of Table 3, an enzyme-labelled second antibody is used. This second antibody is an antibody to IgG, etc. so that if a sample containing an antibody which is to be measured is serum or plasma, it binds to immunoglobulin in blood. However, since unnecessary protein in the sample solution can be previously removed by the first washing solution as in the third embodiment, the reaction is not inhibited by unnecessary immunoglobulin even when a sandwich type assay using the second antibody as mentioned above is applied to. In this respect, this is greatly different from the combination of No. 5 in Table 1 of the first embodiment in which a sample to be measured is limited only to that which does not contain unnecessary immunoglobulin. That is, according to the third embodiment, the sandwich method using the second antibody can be easily applied to for assaying an amount of an antibody in a blood sample such as serum, blood plasma and whole blood.

### EXAMPLES

In the following, the present invention will be explained in more detail by referring to Examples.

### Example 1

To DD100 (trade name) filter paper produced by Whatman Co. were dotted 40 µl of a 0.2 M phosphate buffer (pH = 7.5) at the position 3 mm from the bottom end as a reagent zone inhibiting light emission and 40 µl (1 µg/ml) of an alkaliphosphatase-labelled anti-human CRP mouse IgG solution at the position 40 mm from the bottom end as a labelling reagent zone, and further 40 µl of an anti-human CRP mouse IgG-bound latex at the position 60 mm from the bottom end as a detecting zone and the filter paper was dried to obtain a test strip for measuring CRP. 50 µl of a specimen was dotted to at the position 50 mm from the bottom end of this test strip which was a specimen dotting zone and a layer of a substrate solution containing 0.2 % of AMPPD was broken to develop the substrate solution for 5 minutes by capillarity. Thereafter, it was exposed to a Polaroid instant photograph 615 printing paper (trade name, available from Polaroid Co.) for 2 seconds to obtain an emitted area. The results are shown in Table 4. In Table 4, the area ratio if an area ratio in which a light emitted area per 1 mm² as 100.

**Table 4**

| Measurement of CRP | |
|---|---|
| CRP Concentration (ng/ml) | Area ratio of emitted portion |
| 0 | 0 |
| 0.37 | 200 |
| 0.75 | 800 |
| 1.5 | 5600 |
| 3.0 | 18000 |

### Example 2

To DD100 (trade name) filter paper produced by Whatman Co. were dotted 40 µl of a 0.1 M oxalate buffer (pH = 5.0) at the position 3 mm from the bottom end as a reagent zone inhibiting light emission and 40 µl (1 µg/ml) of an alkaliphosphatase-labelled anti-human CRP mouse IgG solution at the position 40 mm from the bottom end as a labelling reagent zone, and further 40 µl of an anti-human CRP mouse IgG-bound latex at the position 60 mm from the bottom end as a detecting zone and the filter paper was dried to obtain a test strip for measuring CRP. 50 µl of a specimen was dotted to at the position 50 mm from the bottom end of this test strip which was a specimen dotting zone and a layer of a substrate solution containing 0.2 % of AMPPD was broken to develop the substrate solution for 5 minutes by capillarity. Thereafter, emitted light at the portion 55 mm to 65 mm from the bottom end was obtained as an integrated value for 5 seconds. The results are shown in Table 5.

**Table 5**

| Measurement of CRP | |
|---|---|
| CRP Concentration (ng/ml) | Count/5 seconds |
| 0 | 350 |
| 5 | 1850 |
| 10 | 3420 |
| 20 | 6260 |

### Example 3

To DD100 (trade name) filter paper produced by Whatman Co. were dotted 40 µl of a 0.2 M phosphate buffer (pH = 7.5) at the position 3 mm from the bottom end as a reagent zone inhibiting light emission and 40 µl (1 µg/ml) of a mixed solution of an alkaliphosphatase-labelled anti-human CRP mouse IgG and an alkaliphosphatase-labelled anti-human hemoglobin goat IgG at the position 40 mm from the bottom end as a labelling reagent zone. Then, to the filter paper were further dotted 40 µl of an anti-human CRP mouse IgG-bound latex at the position 60 mm from the bottom end as a detecting zone and 40 µl of an anti-human hemoglobin goat IgG-bound latex at the position 70 mm from the bottom end as a detecting zone, and the filter paper was freeze-dried to obtain a test strip for measuring CRP and hemoglobin. 50 µl of a specimen was dotted to at the position 50 mm from the bottom end of this test strip which was a specimen dotting zone and a layer of a substrate solution containing 0.2 % of AMPPD was broken to develop the substrate solution for 5 minutes by capillarity. Thereafter, luminescences at respective detecting zones containing the anti-human CRP mouse IgG-bound latex and the anti-human hemoglobin goat IgG-bound latex were counted for 5 minutes by an Aloka lumino meter (trade name, produced by Aloka Co., Ltd.). The results are shown in Table 6.

**Table 6**

| Simultaneous measurement of CRP and hemoglobin | | |
|---|---|---|
| Specimen | CRP (count/5 sec) | Hemoglobin (count/5 sec) |
| A | 540 | 350 |
| B | 1420 | 1050 |
| C | 180 | 1040 |
| D | 1900 | 1320 |

The test strip for immunoassay with simple and ease of the present invention uses a light emission substrate and has a reagent zone which inhibits light emission and a reagent zone which initiate light emission during measurement. If the test strip is used, an amount of an object to be measured in a specimen can be detected within 2 minutes to 10 minutes whereby the present invention can provide a method which can give a result within a shorter time easily than that of the prior art method. Further, according to the test strip of the present invention, plural number of items to be measured can be effected with one test strip simultaneously.

### Example 4

By using CRP as an antigen to be measured, an immunoassay device to which one step sandwich method had been applied was prepared. The constitutions of which were the same as those of Fig. 3.

To a glass fiber filter paper (DD100, trade name, produced by Whatman Co.) having a width of 1 cm and a length of 15 cm was dotted 40 µl of 1 µg/ml of a POD labelled-anti-human CRP mouse antibody solution (75 mM of ascorbic acid was contained as an enzyme inhibitor) at the position 27 mm from the bottom end thereof (this position becomes a labelling reagent zone). In the same manner, 40 µl of a 1 % suspension of an anti-human CRP mouse antibody-bound latex was dotted at the position 45 mm from the bottom end of the filter paper (this position becomes a detecting zone; the anti-human CRP mouse antibody herein used is an antibody which admits an epitope different from the anti-human CRP mouse antibody which was labelled by POD). This filter paper was freeze-dried to form a labelling reagent zone 16 and a detecting zone 20 to a filter paper matrix 10. In a bag made of an aluminum foil was charged 700 µl of a solution containing 0.05 % H₂O₂ and 0.1 % TMB (3,3',5,5'-tetramethylbenzidine, and the bag was placed at the position 5 mm from the bottom end of the filter paper 10 to make a substrate solution reservoir 12. A bag made of an aluminum foil charged therein 150 µl of PBS (10 mM phosphate buffer physiological saline solution) was placed at the position 35 mm from the bottom end of the filter paper 10 to make a washing solution reservoir 22. At the other end of the filter paper matrix 10, a cellulose filter paper (5 mm in width, 20 mm in length and 2 mm in thickness) was laminated to make a water absorption promoting member 14.

By determining the position 40 mm from the bottom end of the filter paper matrix 10 as a specimen dotting zone 18, 50 µl of a human CRP solution with known concentration was dotted to the device. Then, the washing solution reservoir 22 was broken by pressing with fingers to absorb the washing solution to the filter paper matrix 10. Subsequently, the substrate solution reservoir 12 was broken by pressing to absorb the substrate solution to the filter paper matrix 10 and to develop by capillarity. Coloring degree at the detecting zone 20 was measured by a color difference meter (CR 221, trade name, manufactured by Minoruta Co.) after 10 minutes. The results are shown in Fig. 6.

### Comparative example 1

In the same manner as in Example 4 except for not providing the washing solution reservoir 22, an immunoassay device was prepared as a comparative sample to Example 4.

By using this device, the same operations were carried out in the same manner as in Example 4. The results are also shown in Fig. 6.

As shown in Fig. 6, in Comparative example 1 which did not carry out washing operation by a washing solution reservoir 22, diphasic property with a CRP concentration of 500 ng/ml was appeared and the prozone phenomenon was observed. On the other hand, in Example 1 to which the washing solution reservoir 22 was provided and washing operation was carried out, such a prozone phenomenon was not observed and good linearity was shown until CRP concentration of 5000 ng/ml at which the measured value reaches to a plateau. This means that not only the concentration range which can be measured by the assay device of the present invention is wide but also an amount of an antigen to be measured is not measured as an erroneously low concentration even when a concentration of an antigen to be measured is extremely high.

### Example 5

By using CRP as an antigen to be measured, an immunoassay device to which two step sandwich method had been applied was prepared. The constitutions of which were the same as those of Fig. 5.

To a glass fiber filter paper (DD100, trade name, produced by Whatman Co.) having a width of 1 cm and a length of 15 cm was dotted 40 µl of 1 µg/ml of an alkaliphosphatase-labelled-anti-human CRP mouse antibody solution (50 mM of Tris-HCl buffer solution; pH 7.0) at the position 27 mm from the bottom end thereof (this position becomes a labelling reagent zone). In the same manner, 40 µl of a 1 % suspension of an anti-human CRP mouse antibody-bound latex was dotted at the position 45 mm from the bottom end of the filter paper (this position becomes a detecting zone; the anti-human CRP mouse antibody herein used is an antibody which admits an epitope different from the anti-human CRP mouse antibody which was labelled by alkaliphosphatase). This filter paper was freeze-dried to form a labelling reagent zone 16 and a detecting zone 20 to a filter paper matrix 10. In a bag made of an aluminum foil was charged 700 µl of a solution containing 0.2 % BCIP (5-bromo-4-chloro-3-indolylphosphoric acid) and 0.1 % nitrotetrazolium blue, and the bag was placed at the position 5 mm from the bottom end of the filter paper 10 to make a substrate solution reservoir 12. A bag made of an aluminum foil charged therein 150 µl of 10 mM PBS was placed at the position 35 mm from the bottom end of the filter paper 10 to make a first washing solution reservoir 26. Also, a bag made of an aluminum foil charged therein 100 µl of 10 mM PBS was placed at the position 25 mm from the bottom end of the filter paper 10 to make a second washing solution reservoir 28. At the other end of the filter paper matrix 10, a water absorption promoting member 14 was provided in the same manner as in Example 4.

By determining the position 40 mm from the bottom end of the filter paper matrix 10 of the immunoassay device of Example 5 thus prepared as a specimen dotting zone 18, 50 µl of a human CRP solution with known concentration was dotted thereto. Then, the first washing solution reservoir 26 was broken by pressing with fingers to absorb the first washing solution to the filter paper matrix 10. Subsequently, the second washing solution reservoir 28 was broken by pressing to absorb the second washing solution to the filter paper matrix 10. Subsequently, the substrate solution reservoir 12 was broken by pressing to absorb and develop the substrate solution to the filter paper matrix 10. Coloring degree at the detecting zone 20 was measured by a color difference meter (CR 221, trade name, manufactured by Minoruta Co.) after 10 minutes.

As shown in Fig. 7, the immunoassay device of Example 5 to which two step sandwich method according to stepwise reaction is applied by providing two washing solution reservoirs showed excellent linearity.

### Example 6

By using an antigen of a human adult T-cell leukemia virus (HTLV-1) as an antibody to be measured, an immunoassay device to which two step sandwich method using a second antibody had been applied was prepared. The constitutions of which were the same as those of Fig. 5 and combination of reagents in respective zones corresponds to that of No. 5) in Table 3.

An HTLV-1 antigen-bound latex was prepared as shown below. With 200 µl of a 2.5 % latex suspension was mixed 800 µl of 10 µg/ml of HTLV-1 antigen solution (10 mM acetate buffer: pH 6.0) and the mixture was stirred at room temperature for 18 hours. This suspension was centrifuged (2,500 rpm for 5 minutes) to obtain pellets of the latex. These pellets were suspended again in 2 ml of a 1 % BSA solution (50 mM phosphate buffer solution; pH 6.0) and centrifuged in the same manner. This washing operation was repeated further three times and the final material was suspended in 0.5 ml of the above BSA solution to obtain a 1 % suspension of HTLV-1 antigen-bound latex.

40 µl of the HTLV-1 antigen-bound latex suspension was dotted to a glass fiber filter paper (DD100, trade name, produced by Whatman Co.) having a width of 1 cm and a length of 15 cm (this position becomes a detecting zone) at the position 75 mm from the bottom end thereof.

In the same manner, 40 µl of a solution (50 mM Tris-HCl buffer; pH 7.0) of 100 ng/ml alkaliphosphatase-labelled anti-human IgG mouse antibody (second antibody) was dotted at the position 27 mm from the bottom end of the filter paper (this position becomes a labelling reagent zone).

This filter paper was freeze-dried to form a labelling reagent zone 16 and a detecting zone 20 to a filter paper matrix 10. In a bag made of an aluminum foil was charged 900 µl of a solution containing 0.2 % AMPPD (3-(2'-spiroadamantan)-4-methoxy-4-(3"-phosphoryloxy)phenyl-1,2-dioxetane) disodium salt, and the bag was placed at the position 5 mm from the bottom end of the filter paper 10 to make a substrate solution reservoir 12. A bag made of an aluminum foil charged therein 150 µl of 10 mM PBS was placed at the position 50 mm from the bottom end of the filter paper 10 to make a first washing solution reservoir 26. Also, a bag made of an aluminum foil charged therein 100 µl of 10 mM PBS was placed at the position 20 mm from the bottom end of the filter paper 10 to make a second washing solution reservoir 28. At the other end of the filter paper matrix 10, a water absorption promoting member 14 was provided in the same manner as in Example 4.

By determining the position 65 mm from the bottom end of the filter paper matrix 10 of the immunoassay device of Example 5 thus prepared as a specimen dotting zone 18, 50 µl of human serum samples which are HTLV-1 antibody negative, weakly positive and strongly positive and diluted to 50-fold with a buffer containing 20 mM phosphate (pH 7.0) and 20 % NGS (normal goat serum) were each dotted thereto. Then, the first washing solution reservoir 26 was broken by pressing with fingers to absorb the first washing solution to the filter paper matrix 10. Subsequently, the second washing solution reservoir 28 was broken by pressing to absorb the second washing solution to the filter paper matrix 10. Subsequently, the substrate solution reservoir 12 was broken by pressing to absorb and develop the substrate solution to the filter paper matrix 10. After 10 minutes, an instant photograph 615 printing paper (trade name) available from Polaroid Co. was covered over the detecting zone 20 and exposed for one minute, and an emitted image was measured by a color difference meter (CR 221, trade name, manufactured by Minoruta Co.). Measurement was done with duplicate. The reflected light measured values measured by a color difference meter were shown in Table 7.

In the same manner as in Example 6 except for not providing the first washing solution reservoir 26, an immunoassay device was prepared as a control sample to Example 6, which has the same constitution as that of Example 1.

By using this device, the same operations were carried out in the same manner as in Example 6. The results are also shown in Table 7.

**Table 7**

| HTLV-1 antibody sample | Reflected light dose | |
|---|---|---|
| | Example 6 | Example 1 |
| Negative 1 | 29.1 | 26.4 |
| 2 | 27.3 | 26.6 |
| Weakly positive 1 | 44.8 | 27.5 |
| 2 | 44.7 | 26.8 |
| Strongly positive 1 | 73.1 | 28.8 |
| 2 | 73.0 | 26.7 |

As shown in Table 7, by providing the first washing reservoir 26, HTLV-1 antibody can be detected.

The above results can be analyzed as shown below.

The immunoassay device of Example 1 is not provided a first washing solution reservoir 26. Thus, an antibody to be measured (i.e. anti-HTLV-1 human antibody) contacts with a second antibody (i.e. anti-human IgG mouse antibody) which is transferred to by a washing solution supplied from a second washing solution reservoir. If a sample solution containing an antibody to be measured does not contain any human IgG other than the antibody to be measured, there is no problem. The antibody to be measured binds to an enzyme-labelled second antibody, and further transferred to a detecting zone 20 and binds to an immobilized antigen. However, when the sample solution containing an antibody to be measured is a human blood sample such as serum, blood plasma or whole blood, the second antibody binds not only to an antibody to be measured but also to human IgG so that when the second antibody is passed to the specimen dotting zone 18, it binds to IgG in blood which is present with an amount far larger than that of the antibody to be measured. As the results, an amount of the second antibody which binds to the aimed antibody to be measured remarkable decreases.

To the contrary, in the immunoassay device of Example 6, only a sample to be measured is firstly transferred to a detecting zone by a washing solution from a first washing solution reservoir 26. Thus, an antibody to be measured alone binds to an immobilized antigen and the other unnecessary IgG is washed out and removed from the detecting zone by a washing solution which is still subsequently supplied to. Accordingly, an enzyme-labelled antibody which is thereafter transferred by a washing solution supplied from a second washing solution reservoir can bind to the antibody to be measured without being consumed by unnecessary IgG.

Thus, it can be shown that the immunoassay device having two washing solution reservoir of the embodiment of the present invention can be applied to an assay method using a second antibody.

## Claims

1. An immunoassay device which comprises at least one washing solution reservoir for washing and for preventing interference between respective reagents, said reservoir being provided on a matrix which can transfer a solution by capillarity.

2. The device according to claim 1, wherein a substrate solution reservoir is provided at one end of the matrix and an enzyme-labelling reagent zone, a specimen dotting zone and a detecting zone are provided sequentially in said matrix; and the washing solution reservoir is provided on the matrix at a position upstream, with respect to the direction of solution transfer, of the enzyme-labelling reagent zone and downstream of the substrate solution reservoir.

3. The device according to claim 1, wherein a substrate solution reservoir is provided at one end of the matrix and an enzyme-labelling reagent zone, a specimen dotting zone and a detecting zone are provided sequentially in said matrix; and the washing solution reservoir is provided on the matrix between the enzyme-labelling reagent zone and the specimen dotting zone.

4. The device according to claim 1, wherein a substrate solution reservoir is provided at one end of the matrix and an enzyme-labelling reagent zone, a specimen dotting zone and a detecting zone are provided sequentially in said matrix; and a first washing solution reservoir is provided on the matrix at a position downstream, with respect of the direction of solution transfer, of the enzyme-labelling reagent zone and upstream of the specimen dotting zone and a second washing solution reservoir is provided on the matrix at a position upstream, with respect to the direction of solution transfer, of the enzyme-labelling reagent zone and downstream of the substrate solution reservoir.

5. An immunoassay device according to claim 1, which comprises
(a) a matrix which can transfer a solution by capillarity,
(b) a specimen dotting zone provided in the above matrix, to which a sample solution containing an antigen to be tested is dotted,
(c) a labelling reagent zone containing an antigen or an antibody which is labelled by a labelling enzyme and provided in the matrix at a position separate from the specimen dotting zone,
(d) a detecting zone containing an immobilized antibody and provided in the matrix at a position downstream, with respect to the direction of solution transfer, of the specimen dotting zone and the labelling reagent zone,
(e) a substrate solution reservoir containing a substrate of the labelling enzyme and provided on the matrix at a position upstream, with respect to the direction of solution transfer, of the specimen dotting zone and the labelling reagent zone, and
(f) a washing solution reservoir provided on the matrix at a position upstream, with respect to the direction of solution transfer, of the labelling reagent zone and downstream of the substrate solution reservoir.

6. An immunoassay device according to claim 1, which comprises
(a) a matrix which can transfer a solution by capillarity,
(b) a specimen dotting zone provided in the above matrix, to which a sample solution containing an antibody to be tested is dotted,
(c) a labelling reagent zone containing either an antigen, an antibody or a second antibody which is labelled by a labelling enzyme and provided in the matrix at a position separate from the specimen dotting zone,
(d) a detecting zone containing an immobilized antigen and provided in the matrix at a position downstream, with respect to the direction of solution transfer, of the specimen dotting zone and the labelling reagent zone,
(e) a substrate solution reservoir containing a substrate of the labelling enzyme and provided on the matrix at a position upstream, with respect to the direction of solution transfer, of the specimen dotting zone and the labelling reagent zone, and
(f) a washing solution reservoir provided on the matrix at a position upstream, with respect to the direction of solution transfer, of the labelling reagent zone and downstream of the substrate solution reservoir.

7. An immunoassay device according to claim 1, which comprises
(a) a matrix which can transfer a solution by capillarity,
(b) a specimen dotting zone provided in the above matrix, to which a sample solution containing an antigen to be tested is dotted,
(c) a labelling reagent zone containing an antigen or an antibody which is labelled by a labelling enzyme and provided in the matrix at a position separate from the specimen dotting zone,
(d) a detecting zone containing an immobilized antibody and provided in the matrix at a position downstream, with respect to the direction of solution transfer, of the specimen dotting zone and the labelling reagent zone,
(e) a substrate solution reservoir containing a substrate of the labelling enzyme and provided on the matrix at a position upstream, with respect to the direction of solution transfer, of the specimen dotting zone and the labelling reagent zone, and
(f) a washing solution reservoir provided on the matrix at a position between the labelling reagent zone and the specimen dotting zone.

8. An immunoassay device according to claim 1, which comprises
(a) a matrix which can transfer a solution by capillarity,
(b) a specimen dotting zone provided in the above matrix, to which a sample solution containing an antibody to be tested is dotted,
(c) a labelling reagent zone containing either an antigen, an antibody or a second antibody which is labelled by a labelling enzyme and provided in the matrix at a position separate from the specimen dotting zone,
(d) a detecting zone containing an immobilized antigen and provided in the matrix at a position downstream, with respect to the direction of solution transfer, of the specimen dotting zone and the labelling reagent zone,
(e) a substrate solution reservoir containing a substrate of the labelling enzyme and provided on the matrix at a position upstream, with respect to the direction of solution transfer, of the specimen dotting zone and the labelling reagent zone, and
(f) a washing solution reservoir provided on the matrix at a position between the labelling reagent zone and the specimen dotting zone.

9. An immunoassay device according to claim 1, which comprises
(a) a matrix which can transfer a solution by capillarity,
(b) a specimen dotting zone provided in the above matrix, to which a sample solution containing an antigen to be tested is dotted,
(c) a labelling reagent zone containing an antigen or an antibody which is labelled by a labelling enzyme and provided in the matrix at a position upstream, with respect to the direction of solution transfer, of the specimen dotting zone,
(d) a detecting zone containing an immobilized antibody and provided in the matrix at a position downstream, with respect to the direction of solution transfer, of the specimen dotting zone,
(e) a substrate solution reservoir containing a substrate of the labelling enzyme and provided on the matrix at a position upstream, with respect to the direction of solution transfer, of the labelling reagent zone,
(f) a first washing solution reservoir provided on the matrix at a position downstream, with respect to the direction of solution transfer, of the labelling reagent zone and upstream of the specimen dotting zone, and
(g) a second washing solution reservoir provided on the matrix at a position upstream, with respect to the direction of solution transfer, of the labelling reagent zone and downstream of the substrate solution reservoir.

10. An immunoassay device according to claim 1, which comprises
(a) a matrix which can transfer a solution by capillarity,
(b) a specimen dotting zone provided in the above matrix, to which a sample solution containing an antibody to be tested is dotted,
(c) a labelling reagent zone containing either an antigen, an antibody or a second antibody which is labelled by a labelling enzyme and provided in the matrix at a position upstream, with respect to the direction of solution transfer, of the specimen dotting zone,
(d) a detecting zone containing an immobilized antigen and provided in the matrix at a position downstream, with respect to the direction of solution transfer, of the specimen dotting zone,
(e) a substrate solution reservoir containing a substrate of the labelling enzyme and provided on the matrix at a position upstream, with respect to the direction of solution transfer, of the labelling reagent zone,
(f) a first washing solution reservoir provided on the matrix at a position downstream, with respect to the direction of solution transfer, of the labelling reagent zone and upstream of the specimen dotting zone, and
(g) a second washing solution reservoir provided on the matrix at a position upstream, with respect to the direction of solution transfer, of the labelling reagent zone and downstream of the substrate solution reservoir.

11. An immunoassay method which comprises using a matrix which can transfer a solution by capillarity, said matrix having provided thereon at least one washing solution reservoir for washing and for preventing interference between respective reagents.

12. An immunoassay method according to claim 11, which comprises using an immunoassay device being provided with a substrate solution reservoir at one end of a matrix capable of transferring a solution by capillarity and being sequentially provided with an enzyme-labelling reagent zone, a specimen dotting zone and a detecting zone in the matrix; and being provided with a washing solution reservoir on the matrix at a position upstream, with respect to the direction of solution transfer, of the enzyme-labelling reagent zone and downstream of the substrate solution reservoir,
dotting a sample solution to the specimen dotting zone, supplying a washing solution from the washing solution reservoir to the matrix, supplying a substrate solution from the substrate solution reservoir to the matrix and detecting a labelling enzyme which is immobilized directly or indirectly to the detecting zone with an amount which depends on an amount of a material to be measured in the sample solution.

13. An immunoassay method according to claim 11, which comprises using an immunoassay device being provided with a substrate solution reservoir at one end of a matrix capable of transferring a solution by capillarity and being sequentially provided with an enzyme-labelling reagent zone, a specimen dotting zone and a detecting zone in the matrix; and being provided with a washing solution reservoir on the matrix between the enzyme-labelling reagent zone and the specimen dotting zone,
dotting a sample solution to the specimen dotting zone, supplying a washing solution from the washing solution reservoir to the matrix, supplying a substrate solution from the substrate solution reservoir to the matrix and detecting a labelling enzyme which is immobilized directly or indirectly to the detecting zone with an amount which depends on an amount of a material to be measured in the sample solution.

14. An immunoassay method according to claim 11, which comprises using an immunoassay device being provided with a substrate solution reservoir at one end of a matrix capable of transferring a solution by capillarity and being sequentially provided with an enzyme-labelling reagent zone, a specimen dotting zone and a detecting zone in the matrix; and being provided with a first washing solution reservoir on the matrix at a position downstream, with respect to the direction of solution transfer, of the enzyme-labelling reagent zone and upstream of the specimen dotting zone and a second washing solution reservoir on the matrix at a position upstream, with respect to the direction of solution transfer, of the enzyme-labelling reagent zone and downstream of the substrate solution reservoir,
dotting a sample solution to the specimen dotting zone, supplying a first washing solution from the first washing solution reservoir to the matrix, supplying a second washing solution from the second washing solution reservoir to the matrix, supplying a substrate solution from the substrate solution reservoir to the matrix and detecting a labelling enzyme which is immobilized directly or indirectly to the detecting zone with an amount which depends on an amount of a material to be measured in the sample solution.

## Patentansprüche

1. Immuntestvorrichtung, welche mindestens ein Waschlösungsreservoir zum Waschen und Vermeiden von Störungen zwischen den entsprechenden Reagenzien enthält, wobei das Reservoir auf einer Matrix vorgesehen ist, welche eine Lösung durch Kapillarkraft transferieren kann.

2. Vorrichtung nach Anspruch 1, worin das Substratlösungsreservoir an einem Ende der Matrix vorgesehen ist, und eine Enzym-Markierungsreagenzzone, eine Probenauftragszone und eine Nachweiszone der Reihe nach an der Matrix vorgesehen sind; und das Waschlösungsreservoir auf der Matrix an einer Position in Laufrichtung oberhalb der Enzym-Markierungsreagenzzone und abwärts des Substratlösungsreservoirs vorgesehen ist.

3. Vorrichtung nach Anspruch 1, worin das Substratlösungsreservoir an einem Ende der Matrix vorgesehen ist, und eine Enzym-Markierungsreagenzzone, eine Probenauftragszone und eine Nachweiszone der Reihe nach an auf der Matrix vorgesehen sind; und das Waschlösungsreservoir auf der Matrix zwischen der Enzym-Markierungsreagenzzone und der Probenauftragszone vorgesehen ist.

4. Vorrichtung nach Anspruch 1, worin das Substratlösungsreservoir an einem Ende der Matrix vorgesehen ist, und eine Enzym-Markierungsreagenzzone, eine Probenauftragszone und eine Nachweiszone der Reihe nach auf der Matrix vorgesehen sind; und ein erstes Waschlösungsreservoir auf der Matrix an einer Position in Laufrichtung unterhalb der Enzym-Markierungsreagenzzone und aufwärts der Probenauftragszone vorgesehen ist, und ein zweites Waschlösungsreservoir auf der Matrix an einer Position in Laufrichtung oberhalb der Enzym-Markierungsreagenzzone und unterhalb des Substratlösungsreservoirs vorgesehen ist.

5. Immuntestvorrichtung nach Anspruch 1, welche umfaßt:
(a) eine Matrix, welche eine Lösung durch Kapillarkraft transferieren kann,
(b) eine Probenauftragszone, vorgesehen in obiger Matrix, auf die eine Probenlösung, welche das zu testende Antigen enthält, aufgetragen wird,
(c) eine Markierungsreagenzzone, enthaltend ein Antigen oder einen Antikörper, der durch Markierungsenzym markiert ist und in der Matrix an einer Position von der Probenauftragszone getrennten Position vorgesehen ist,
(d) eine Nachweiszone, enthaltend einen immobilisierten Antikörper und vorgesehen in der Matrix an einer Position in Laufrichtung unterhalb der Probenauftragszone und der Markierungsreagenzzone,
(e) ein Substratlösungsreservoir, enthaltend ein Substrat des Markierungsenzyms, und vorgesehen auf der Matrix an einer Position in Laufrichtung oberhalb der Probenauftragszone und der Markierungsreagenzzone, und
(f) ein Waschlösungsreservoir, vorgesehen auf der Matrix an einer Position in Laufrichtung oberhalb der Markierungsreagenzzone und abwärts des Substratlösungsreservoirs.

6. Immuntestvorrichtung nach Anspruch 1, welche umfaßt:
(a) eine Matrix, welche eine Lösung durch Kapillarkraft transferieren kann,
(b) eine Probenauftragszone, die auf der obigen Matrix vorgesehen ist, auf die eine einen zu testenden Antikörper enthaltende Probenlösung aufgetragen wird,
(c) eine Markierungsreagenzzone, die entweder ein Antigen, einen Antikörper oder einen zweiten Antikörper enthält, der durch ein Enzym markiert ist und in der Matrix an einer von der Probenauftragszone abgetrennten Position vorgesehen ist,
(d) eine Nachweiszone, enthaltend ein immobilisiertes Antigen und vorgesehen in der Matrix an einer Position in Laufrichtung unterhalb der Probenauftragszone und der Markierungsreagenzzone,
(e) ein Substratlösungsreservoir, enthaltend ein Substrat aus einem Markierungsenzym und vorgesehen in der Matrix an einer Position in Laufrichtung oberhalb der Probenauftragszone und der Markierungsreagenzzone, und
(f) ein Waschlösungsreservoir, vorgesehen auf der Matrix an einer Position in Laufrichtung oberhalb der Markierungsreagenzzone und abwärts des Substratlösungsreservoirs.

7. Immuntestvorrichtung nach Anspruch 1, welche enthält:
(a) eine Matrix, welche eine Lösung durch Kapillarkraft transferieren kann,
(b) eine Probenauftragszone, vorgesehen auf der Matrix, auf die eine ein zu testendes Antigen enthaltende Probenlösung aufgetragen wird,
(c) eine Markierungsreagenzzone, enthaltend ein Antigen oder einen Antikörper, der mit einem Markierungsenzym markiert ist und in der Matrix an einer unterschiedlichen Position von der Probenauftragszone vorgesehen ist,
(d) eine Nachweiszone, enthaltend einen immobilisierten Antikörper und vorgesehen auf der Matrix an einer Position in Laufrichtung unterhalb der Probenauftragszone und der Markierungsreagenzzone,
(e) ein Substratlösungsreservoir, enthaltend ein Substrat aus einem Markierungsenzym und vorgesehen auf der Matrix an einer Position in Laufrichtung oberhalb der Probenauftragszone und der Markierungsreagenzzone, und
(f) ein Waschlösungsreservoir, vorgesehen auf der Matrix an einer Position zwischen der Markierungsreagenzzone und der Probenauftragszone.

8. Immuntestvorrichtung nach Anspruch 1, welche umfaßt:
(a) eine Matrix, welche eine Lösung durch Kapillarkraft transferieren kann,
(b) eine Probenauftragszone, vorgesehen auf der obigen Matrix, auf die eine den zu testenden Antikörper enthaltende Probenlösung aufgetragen wird,
(c) eine Markierungsreagenzzone, die entweder ein Antigen, einen Antikörper oder einen zweiten Antikörper enthält, der mit einem Markierungsenzym markiert ist, und an einer getrennten Position von der Probenauftragszone vorgesehen ist,
(d) eine Nachweiszone, enthaltend ein immobilisiertes Antigen und vorgesehen auf der Matrix an einer Position in Laufrichtung unterhalb der Probenauftragszone und der Markierungsreagenzzone,
(e) ein Substratlösungsreservoir, enthaltend ein Substrat aus einem Markierungsenzym und vorgesehen auf der Matrix an einer Position in Laufrichtung oberhalb der Probenauftragszone und der Markierungsreagenzzone, und
(f) ein Waschlösungsreservoir, vorgesehen auf der Matrix an einer Position zwischen der Markierungsreagenzzone und der Probenauftragszone.

9. Immuntestvorrichtung nach Anspruch 1, welche umfaßt:
(a) eine Matrix, die eine Lösung durch Kapillarkraft transferieren kann,
(b) eine in der obigen Matrix vorgesehene Probenauftragszone, auf die eine ein zu testendes Antigen enthaltende Probenlösung aufgetragen wird,
(c) eine Markierungsreagenzzone, die ein Antigen oder einen Antikörper enthält, der mit einem Markierungsenzym markiert ist, und auf der Matrix an einer Position in Laufrichtung oberhalb der Probenauftragszone angeordnet ist,
(d) eine Nachweiszone, enthaltend einen immobilisierten Antikörper und vorgesehen an einer Position in Laufrichtung unterhalb der Probenauftragszone in der Matrix,
(e) ein Substratlösungsreservoir, enthaltend ein Substrat des Markierungsenzyms und vorgesehen in der Matrix an einer Position in Laufrichtung oberhalb der Markierungsreagenzzone,
(f) ein erstes Waschlösungsreservoir, vorgesehen auf der Matrix an einer Position in Laufrichtung unterhalb der Markierungsreagenzzone und oberhalb der Probenauftragszone, und
g) ein zweites Waschlösungsreservoir, vorgesehen auf der Matrix an einer Position in Laufrichtung oberhalb der Markierungsreagenzzone und unterhalb des Substratlösungsreservoirs.

10. Immuntestvorrichtung nach Anspruch 1, welche umfaßt:
(a) eine Matrix, welche eine Lösung durch Kapillarkraft transferieren kann,
(b) eine Probenauftragszone, vorgesehen auf obiger Matrix, auf die eine einen zu testenden Antikörper enthaltende Probenlösung aufgetropft wird,
(c) eine Markierungsreagenzzone, enthaltend entweder ein Antigen, einen Antikörper oder einen zweiten Antikörper, der durch das Markierungsenzym markiert ist, und vorgesehen in der Matrix an einer Position in Laufrichtung oberhalb der Probenauftragszone,
(d) eine Nachweiszone, enthaltend ein immobilisiertes Antigen, und vorgesehen in der Matrix an einer Position in Laufrichtung unterhalb der Probenauftragszone,
(e) ein Substratlösungsreservoir, enthaltend ein Substrat eines Markierungsenzyms und vorgesehen auf der Matrix an einer Position in Laufrichtung oberhalb der Markierungsreagenzzone,
(f) ein erstes Waschlösungsreservoir, vorgesehen auf der Matrix an einer Position in Laufrichtung unterhalb der Markierungsreagenzzone und oberhalb der Probenauftragszone, und
g) ein zweites Waschlösungsreservoir, vorgesehen auf der Matrix an einer Position in Laufrichtung oberhalb der Markierungsreagenzzone und unterhalb des Substratlösungsreservoirs.

11. Immuntestverfahren, welches eine Matrix umfaßt, die eine Lösung durch Kapillarkraft transferieren kann, wobei auf der Matrix mindestens ein Waschlösungsreservoir zum Waschen und zum Verhindern von Störungen zwischen den entsprechenden Reagenzien vorgesehen ist.

12. Immuntestverfahren nach Anspruch 11, welches eine Immuntestvorrichtung umfaßt, welche mit einem Substratlösungsreservoir an einem Ende der Matrix ausgestattet ist, das zum Transferieren einer Lösung durch Kapillarkraft in der Lage ist und der Reihe nach mit einer Enzym-Markierungsreagenzzone, einer Probenauftragszone und einer Nachweiszone in der Matrix angeordnet ist, und wobei mit einem Waschlösungsreservoir auf der Matrix an einer Position in Laufrichtung oberhalb der Enzym-markierten Reagenzzone und unterhalb des Substratlösungsreservoirs ausgerüstet ist,
Auftragen der Probenlösung auf die Probenauftragszone, Zufuhr einer Waschlösung aus dem Waschlösungsreservoir auf der Matrix, Zuführen einer Substratlösung aus dem Substratlösungsreservoir auf der Matrix und Nachweis eines markierten Enzyms, welches direkt oder indirekt an die Nachweiszone mit einer Menge immobilisiert ist, die von der Menge des zu messenden Materials in der Probenlösung abhängt.

13. Immuntestverfahren nach Anspruch 11, welches die Verwendung einer Immuntestvorrichtung umfaßt, die mit einem Substratlösungsreservoir an einem Ende der Matrix ausgestattet ist, welche zum Transfer einer Lösung durch Kapillarkraft in der Lage ist und die der Reihe nach vorgesehen ist mit einer Enzym-Markierungsreagenzzone, einer Probenauftragszone und einer Nachweiszone in der Matrix, und die mit einem Waschlösungsreservoir auf der Matrix zwischen der Enzym-Markierungsreagenzzone und der Probenauftragszone vorgesehen ist,
Auftragen einer Probenlösung auf die Probenauftragszone zum Zuführen einer Waschlösung aus dem Waschlösungsreservoir in die Matrix, Zuführen einer Substratlösung auf dem Substratlösungsreservoir in die Matrix und Nachweis eines Markierungsenzyms, welches direkt oder indirekt in der Nachweiszone immobilisiert ist in einer Menge, die von der Menge des zu messenden Materials in der Probenlösung abhängt.

14. Immuntestverfahren nach Anspruch 11, welches eine Immuntestvorrichtung enthält, welche mit einem Substratlösungsreservoir an einem Ende der Matrix ausgestattet ist, das zum Transfer einer Lösung durch Kapillarkraft in der Lage ist und welche der Reihe nach angeordnet ist mit einer Enzym-Markierungszone, einer Probenauftragszone und einer Nachweiszone in der Matrix, und die mit einem Waschlösungsreservoir auf der Matrix an einer Position in Laufrichtung unterhalb der Enzym-Markierungsreagenzzone und oberhalb der Probenauftragszone ausgerüstet ist, und einem zweiten Waschlösungsreservoir auf der Matrix an einer Position in Laufrichtung oberhalb der Enzym-Markierungsreagenzzone und unterhalb des Substratlösungsreservoirs,
Auftragen einer Probenlösung auf die Probenauftragszone, Zuführen einer ersten Waschlösung aus dem ersten Waschlösungsreservoir in die Matrix, Zuführen einer zweiten Waschlösung aus dem zweiten Waschlösungsreservoir in die Matrix, Zuführen einer Substratlösung aus dem Substratlösungsreservoir in die Matrix und Nachweis eines Markierungsenzyms, welches direkt oder indirekt in der Nachweiszone immobilisiert ist in einer Menge, welche von der Menge des in der Probenlösung zu messenden Materials abhängt.

## Revendications

1. Dispositif d'essai immunologique, comprenant au moins un réservoir de solution de lavage pour le lavage et la prévention d'interférences entre des réactifs respectifs, ledit réservoir étant disposé sur une matrice pouvant transférer une solution par capillarité.

2. Dispositif selon la revendication 1, caractérisé en ce que le réservoir de solution de substrat est disposé à une extrémité de la matrice et une zone de réactif de marquage enzymatique, une zone de dépôt ponctuel de prélèvement et une zone de détection sont disposées séquentiellement dans ladite matrice; et le réservoir de solution de lavage est disposé sur la matrice à une position en amont, par rapport à la direction de transfert de solution, de la zone de réactif de marquage enzymatique et en aval du réservoir de solution de substrat.

3. Dispositif selon la revendication 1, caractérisé en ce qu'un réservoir de solution de substrat est disposé à une extrémité de la matrice et une zone de réactif de marquage enzymatique, une zone de dépôt ponctuel de prélèvement et une zone de détection sont disposés séquentiellement dans ladite matrice; et le réservoir de solution de lavage est disposé sur la matrice entre la zone de réactif de marquage enzymatique et la zone de dépôt ponctuel de prélèvement.

4. Dispositif selon la revendication 1, caractérisé en ce qu'un réservoir de solution de substrat est disposé à une extrémité de la matrice et une zone de réactif de marquage enzymatique, une zone de dépôt ponctuel de prélèvement et une zone de détection sont disposées séquentiellement dans ladite matrice; et un premier réservoir de solution de lavage est disposé sur la matrice à une position en aval, par rapport à la direction de transfert de solution, de la zone de réactif de marquage enzymatique et en amont de la zone de dépôt ponctuel de prélèvement et un deuxième réservoir de solution de lavage est prévu sur la matrice à une position en amont, par rapport à la direction de transfert de solution, de la zone de réactif de marquage enzymatique et en aval du réservoir de solution de substrat.

5. Dispositif d'essai immunologique selon la revendication 1, comprenant:
(a) une matrice pouvant transférer une solution par capillarité,
(b) une zone de dépôt ponctuel de prélèvement disposée dans la matrice ci-dessus, sur laquelle on effectue le dépôt ponctuel d'une solution d'échantillon contenant un antigène à tester,
(c) une zone de réactif de marquage contenant un antigène ou un anticorps marqué par une enzyme de marquage et disposée dans la matrice à une position distincte de la zone de dépôt ponctuel de prélèvement,
(d) une zone de détection contenant un anticorps immobilisé et disposée dans la matrice à une position en aval, par rapport à la direction de transfert de solution, de la zone de dépôt ponctuel de prélèvement et de la zone de réactif de marquage,
(e) un réservoir de solution de substrat contenant un substrat de l'enzyme de marquage et disposé sur la matrice à une position en amont, par rapport à la direction de transfert de solution, de la zone de dépôt ponctuel de prélèvement et de la zone de réactif de marquage, et
(f) un réservoir de solution de lavage disposé sur la matrice à une position en amont, par rapport à la direction de transfert de solution, de la zone de réactif de marquage et en aval du réservoir de solution de substrat.

6. Dispositif d'essai immunologique selon la revendication 1, comprenant:
(a) une matrice pouvant transférer une solution par capillarité,
(b) une zone de dépôt ponctuel de prélèvement disposée dans la matrice ci-dessus, sur laquelle on effectue le dépôt ponctuel d'une solution d'échantillon contenant un anticorps à tester,
(c) une zone de réactif de marquage contenant soit un antigène, un anticorps soit un deuxième anticorps marqué par une enzyme de marquage et disposée dans la matrice à une position distincte de la zone de dépôt ponctuel de prélèvement,
(d) une zone de détection contenant un antigène immobilisé et disposée dans la matrice à une position en aval, par rapport à la direction de transfert de solution, de la zone de dépôt ponctuel de prélèvement et de la zone de réactif de marquage,
(e) un réservoir de solution de substrat contenant un substrat de l'enzyme de marquage et disposé sur la matrice à une position en amont, par rapport à la direction de transfert de solution, de la zone de dépôt ponctuel de prélèvement et de la zone de réactif de marquage, et
(f) un réservoir de solution de lavage prévu sur la matrice à une position en amont, par rapport à la direction de transfert de solution, de la zone de réactif de marquage et en aval du réservoir de solution de substrat.

7. Dispositif d'essai immunologique selon la revendication 1, comprenant:
(a) une matrice pouvant transférer une solution par capillarité,
(b) une zone de dépôt ponctuel de prélèvement prévue dans la matrice ci-dessus, sur laquelle on effectue le dépôt ponctuel d'une solution d'échantillon contenant un antigène à tester,
(c) une zone de réactif de marquage contenant un antigène ou un anticorps marqué par une enzyme de marquage et disposée dans la matrice à une position distincte de la zone de dépôt ponctuel de prélèvement,
(d) une zone de détection contenant un anticorps immobilisé et disposée dans la matrice à une position en aval, par rapport à la direction de transfert de solution, de la zone de dépôt ponctuel de prélèvement et de la zone de réactif de marquage,
(e) un réservoir de solution de substrat contenant un substrat de l'enzyme de marquage et disposé sur la matrice à une position en amont, par rapport à la direction de transfert de solution, de la zone de dépôt ponctuel de prélèvement et de la zone de réactif de marquage, et
(f) un réservoir de solution de lavage disposé sur la matrice à une position comprise entre la zone de réactif de marquage et la zone de dépôt ponctuel de prélèvement.

8. Dispositif d'essai immunologique selon la revendication 1, comprenant:
(a) une matrice pouvant transférer une solution par capillarité,
(b) une zone de dépôt ponctuel de prélèvement disposée dans la matrice ci-dessus, sur laquelle on effectue le dépôt ponctuel d'une solution d'échantillon contenant un anticorps à tester,
(c) une zone de réactif de marquage contenant soit un antigène, un anticorps soit un deuxième anticorps marqué par une enzyme de marquage et disposée dans la matrice à une position distincte de la zone de dépôt ponctuel de prélèvement,
(d) une zone de détection contenant un antigène immobilisé et disposée dans la matrice à une position en aval, par rapport à la direction de transfert de solution, de la zone de dépôt ponctuel de prélèvement et de la zone de réactif de marquage,
(e) un réservoir de solution de substrat contenant un substrat de l'enzyme de marquage et disposé sur la matrice à une position en amont, par rapport à la direction de transfert de solution, de la zone de dépôt ponctuel de prélèvement et de la zone de réactif de marquage, et
(f) un réservoir de solution de lavage disposé sur la matrice à une position comprise entre la zone de réactif de marquage et la zone de dépôt ponctuel de prélèvement.

9. Dispositif d'essai immunologique selon la revendication 1, comprenant:
(a) une matrice pouvant transférer une solution par capillarité,
(b) une zone de dépôt ponctuel de prélèvement disposée dans la matrice ci-dessus, sur laquelle on effectue le dépôt ponctuel d'une solution d'échantillon contenant un antigène à tester,
(c) une zone de réactif de marquage contenant un antigène ou un anticorps marqué par une enzyme de marquage et disposée dans la matrice à une position en amont, par rapport à la direction de tranfert de solution, de la zone de dépôt ponctuel de prélèvement,
(d) une zone de détection contenant un anticorps immobilisé et disposée dans la matrice à une position en aval, par rapport à la direction de transfert de solution, de la zone de dépôt ponctuel de prélèvement,
(e) un réservoir de solution de substrat contenant un substrat de l'enzyme de marquage et disposé sur la matrice à une position en amont, par rapport à la direction de transfert de solution, de la zone de réactif de marquage,
(f) un premier réservoir de solution de lavage disposé sur la matrice à une position en aval, par rapport à la direction de transfert de solution, de la zone de réactif de marquage et en amont de la zone de dépôt ponctuel de prélèvement, et
(g) un deuxième réservoir de solution de lavage prévu sur la matrice à une position en amont, par rapport à la direction de transfert de solution, de la zone de réactif de marquage et en aval du réservoir de solution de substrat.

10. Dispositif d'essai immunologique selon la revendication 1, comprenant:
(a) une matrice pouvant transférer une solution par capillarité,
(b) une zone de dépôt ponctuel de prélèvement disposeé dans la matrice ci-dessus, sur laquelle on effectue le dépôt ponctuel d'une solution d'échantillon contenant un anticorps à tester,
(c) une zone de réactif de marquage contenant soit un antigène, un anticorps soit un deuxième anticorps marqué par une enzyme de marquage et disposeé dans la matrice à une position en amont, par rapport à la direction de tranfert de solution, de la zone de dépôt ponctuel de prélèvement,
(d) une zone de détection contenant un anticorps immobilisé et disposeé dans la matrice à une position en aval, par rapport à la direction de transfert de solution, de la zone de dépôt ponctuel de prélèvement,
(e) un réservoir de solution de substrat contenant un substrat de l'enzyme de marquage et disposé sur la matrice à une position en amont, par rapport à la direction de transfert de solution, de la zone de réactif de marquage,
(f) un premier réservoir de solution de lavage disposé sur la matrice à une position en aval, par rapport à la direction de transfert de solution, de la zone de réactif de marquage et en amont de la zone de dépôt ponctuel de prélèvement, et
(g) un deuxième réservoir de solution de lavage disposé sur la matrice à une position en amont, par rapport à la direction de transfert de solution, de la zone de réactif de marquage et en aval du réservoir de solution de substrat.

11. Procédé d'essai immunologique, comprenant l'utilisation d'une matrice pouvant transférer une solution par capillarité, ladite matrice ayant, disposé sur celle-ci, au moins un réservoir de solution de lavage pour le lavage et la prévention d'interférences entre les réactifs respectifs.

12. Procédé d'essai immunologique selon la. revendication 11, comprenant l'utilisation d'un dispositif d'essai immunologique équipé d'un réservoir de solution de substrat à une extrémité d'une matrice susceptible de transférer une solution par capillarité et équipé séquentiellement de la zone de réactif de marquage enzymatique, d'une zone de dépôt ponctuel de prélèvement et d'une zone de détection dans la matrice; et équipé du réservoir de solution de lavage sur la matrice à une position en amont, par rapport à la direction de transfert de solution, de la zone de réactif de marquage enzymatique et en aval du réservoir de solution de substrat,
le dépôt ponctuel d'une solution d'échantillon sur la zone de dépôt ponctuel de prélèvement, l'apport d'une solution de lavage du réservoir de solution de lavage à la matrice, l'apport d'une solution de substrat du réservoir de solution de substrat à la matrice et la détection d'une enzyme de marquage immobilisée directement ou indirectement sur la zone de détection avec une quantité qui dépend d'une quantité d'une matière à mesurer dans la solution d'échantillon.

13. Procédé d'essai immunologique selon la revendication 11, comprenant l'utilisation d'un dispositif d'essai immunologique équipé d'un réservoir de solution de substrat à une extrémité d'une matrice susceptible de transférer une solution par capillarité et équipé séquentiellement d'une zone de réactif de marquage enzymatique, d'une zone de dépôt ponctuel de prélèvement et d'une zone de détection dans la matrice; et équipé d'un réservoir de solution de lavage sur la matrice entre la zone de réactif de marquage enzymatique et la zone de dépôt ponctuel de prélèvement,
le dépôt ponctuel d'une solution d'échantillon sur la zone de dépôt ponctuel de prélèvement, l'apport d'une solution de lavage du réservoir de solution de lavage à la matrice, l'apport d'une solution de substrat du réservoir de solution de substrat à la matrice et la détection d'une enzyme de marquage immobilisée directement ou indirectement sur la zone de détection avec une quantité qui dépend d'une quantité d'une matière à mesurer dans la solution d'échantillon.

14. Procédé d'essai immunologique selon la revendication 11, comprenant l'utilisation d'un dispositif d'essai immunologique équipé d'un réservoir de solution de substrat à une extrémité d'une matrice susceptible de transférer une solution par capillarité et équipé séquentiellement d'une zone de réactif de marquage enzymatique, d'une zone de dépôt ponctuel de prélèvement et d'une zone de détection dans la matrice; et équipé d'un premier réservoir de solution de lavage sur la matrice à une position en aval, par rapport à la direction de transfert de solution, de la zone de réactif de marquage enzymatique et en amont de la zone de dépôt ponctuel de prélèvement et un deuxième réservoir de solution de lavage sur la matrice à une position en amont, par rapport à la direction de transfert de solution, de la zone de réactif de marquage enzymatique et en aval du réservoir de solution de substrat,
le dépôt ponctuel d'une solution d'échantillon sur la zone de dépôt ponctuel de prélèvement, l'apport d'une première solution de lavage du premier réservoir de solution de lavage à la matrice, l'apport d'une deuxième solution de lavage du deuxième réservoir de solution de lavage à la matrice, l'apport d'une solution de substrat du réservoir de solution de substrat à la matrice, et la détection d'une enzyme de marquage immobilisée directement ou indirectement sur la zone de détection avec une quantité qui dépend d'une quantité d'une matière à mesurer dans la solution d'échantillon.
